## Europäisches Patentamt
## European Patent Office
## Office européen des brevets

(19)

(11) Veröffentlichungsnummer: **0 004 600**
**A2**

(12)

# EUROPÄISCHE PATENTANMELDUNG

(21) Anmeldenummer: **79100865.9**

(22) Anmeldetag: **22.03.79**

(51) Int. Cl.²: **A 61 M 1/03**
**F 04 B 43/12**

(30) Priorität: **22.03.78 US 888858**

(43) Veröffentlichungstag der Anmeldung:
**17.10.79 Patentblatt 79/21**

(84) Benannte Vertragsstaaten:
**BE CH DE FR GB IT NL SE**

(71) Anmelder: **HOECHST AKTIENGESELLSCHAFT**
**Zentrale Patentabteilung Postfach 80 03 20**
**D-6230 Frankfurt/Main 80(DE)**

(72) Erfinder: **Savitz, Steven Robert**
**41. Street 3167**
**Astoria, N.Y.(US)**

(72) Erfinder: **Drago, James Alan**
**Brewster Drive 16**
**Brewster, N.Y.(US)**

(54) **Peristaltische Dialysatlösungspumpe.**

(57) Peristaltische Pumpe zum Pumpen einer Dialysatlösung durch einen Dialysatlösungskreislauf in einem Hämodialysesystem. Die Pumpe umfaßt vier Rollen, die auf einer drehbaren Basis befestigt sind, wobei ein verhältnismäßig langer Abschnitt des Kreislaufschlauches unter Spannung so um den Pumpenkopf geführt wird, daß er von mindestens zwei der Rollen eingeschlossen und zusammengedrückt wird. Die Befestigung der Rollen erlaubt eine Längsbewegung der Druckpunkte entlang des Schlauches während der Umdrehung des Pumpenkopfes, wodurch die Dialyselösung im Kreislauf geführt wird. Die vorgeschlagene Pumpe ermöglicht eine im wesentlichen gleichmäßige Strömungsgeschwindigkeit bei Änderungen des negativen Druckes in der Dialysatlösung. Sie kann in einem einteiligen, tragbaren Hämodialysegerät mit Proportionierungsgerät für einmaligen Durchfluß oder mit chargenweiser Zufuhr der Dialyselösung verwendet werden. In dem vorgeschlagenen Proportionierungsgerät werden lösliche Gase in einer Umlaufentlüftungsanlage aus dem für die Dialyselösung verwendeten Wasser entfernt. Die peristaltische Pumpe für die Dialysatlösung wird so im tragbaren Hämodialysegerät befestigt, daß ein schnelles Umschalten von einmaligem Durchfluß auf chargenweise Zufuhr der Lösung und umgekehrt leicht möglich ist.

FIG. 4

**EP 0 004 600 A2**

0004600

HOECHST AKTIENGESELLSCHAFT    HOE 78/F 289

## Peristaltische Dialysatlösungspumpe

Die Erfindung betrifft eine peristaltische Pumpvorrichtung zum Pumpen einer Dialysatlösung durch einen Dialysatlösungs-Flußkreis in einem Hämodialysesystem. Hämodialysesysteme werden im allgemeinen seit mehreren Jahren für die Behandlung von Nierenkrankheiten und Nierenversagen verwendet und haben hohe Effektivität bewiesen, indem sie künstliche Nierenfunktionen für Personen, deren eigenen natürlichen Nieren funktionell beeinträchtigt sind, vorsahen. Bei der Verwendung von Hemodialysesystemen wird Blut, das Abfallstoffe enthält, wie z.B. Harnstoff, Kreatinin, überschüssige elektrolytische Salze und Wasser vom Körper abgezogen und fließen durch einen Dialysator zur indirekten Massenübertragung mit einer wäßrigen Dialysatlösung. Der Dialysator kann einer von vielen bekannten Typen sein mit einem Massenübertragungselement, wie z.B. eine ausgedehnte Fläche einer gummiartigen Membran oder ein Hohlfaserbündel, über welches die Abfallsubstanzen infolge des Konzentrationsgradienten (gelöste Verunreinigungen) oder durch osmotischen Druck (Wasser) vom Blut auf die Dialysatlösung übertragen werden. Vom Dialysator wird das auf diese Weise von Verunreinigungen gesäuberte Blut in den Körper des Patienten zurückgeführt. Die mit Verunreinigungen angereicherte Dialysatlösung des Dialysators wird entweder als Abfallstoff beseitigt oder in einem geschlossenem Kreis ausgehend von einer Dialysatlösungsquelle durch den Dialysator zur erneuerten Massenübertragung vom Blut auf die Dialysatlösung zurückgeführt. Die Dialysatlösung, die in der bekannten Hemodialysebehandlung verwendet wird, ist eine wäßrige Lösung verschiedener ausgewählter Salze, welche die gewünschte elektrolytische Balance mit dem Blut in dem Dialysator vorsehen und auf diese Weise den Verlust von wertvollen Blutbestandteilen an die Dialysatlösung infolge Diffusion und Osmose im Dialysator verhindert.

Im allgemeinen ist es bei der Anwendung der Dialyse häufig wünschenswert, mit einem Dialysesystem so zu arbeiten, daß der Druck in der Dialysatlösung an der einen Seite des Massenübertragungselements des Dialysators z.B. die Dialysatmembran etwas gegenüber dem Druck des Blutes auf der anderen Seite des Dialysatormassenübertragungselements verringert ist (negativer Druck). Diese Art der Anwendung sieht eine Druckdifferenz quer über das Massenübertragungselement des Dialysators vor und wird angewandt, um den Transport von Überschußwasser vom Kreislaufsystem des Patienten auf den Dialysatlösungsstrom mittels osmotischen Drucks zu erleichtern. Die Entfernung von Überschußwasser, im allgemeinen bezeichnet als Ultrafiltration, stellt ein nützliches Mittel zur Entfernung von Wasser aus dem Körper des Patienten, wie das im allgemeinen mit Hilfe der Funktionierenden natürlichen Nieren ausgeführt wird dar.

Der Stand der Technik lehrt bei einem Hämodialysesystem die Verwendung eines Kreislaufes der Dialysatlösung mit einem flexiblen elastischen Schlauchpumpabschnitt, durch welchen die Dialysatlösung mittels einer peristaltischen Pumpvorrichtung, welche mehrere über den Umfang verteilt auf einer drehbaren Basis montierte Rollen aufweist, umzupumpen. In diesem System erstreckt sich der flexible elastische Schlauchpumpenabschnitt unter Spannung um die Rollen, sodaß er mit zumindest zwei der Rollen gleichzeitig im Eingriff steht und zusammengedrückt wird, wobei die Rollen zur Längsbewegung der Kompressionspunkte entlang des Schlauches, während der Drehung der Basis der Pumpe befestigt sind, um die Dialysatlösung durch den Schlauch vorzuschieben.

Eine erst jetzt entwickelte Dialysatlösungs-Peristaltikpumpe der oben beschriebenen Art ist geoffenbart und beansprucht in US-Ser. Nr. 720 672, hinterlegt am 7.9.1976 unter dem Namen "J.T.Hutchisson". Diese Pumpe ist sehr effizient und extrem klein in Größe und Gewicht. Deshalb wurde die bekannte Pumpe in tragbaren Hämodialysesystemen verwendet, für die sie besonders geeignet sind. Mehr im einzelnen weist die Pumpe gemäß Serial No. 720 672 drei auf einer Basis befestigte Rollen auf, wobei jede Rolle einen Durchmesser zwischen 6,35 und 19 mm aufweist und jeweils um einen Winkel von 120° gegenüber der nächsten Rolle versetzt ist, mit einem radialen Abstand zwischen der Rollenachse und der Achse der Basis des Pumpenkopfes, zwischen 12,7 und 31,8 mm.

Die bekannte Pumpe verwendet einen flexiblen elastischen Schlauchpumpenabschnitt mit einer Länge, gemessen der Länge nach entlang des Schlauches zwischen dessen befestigten Enden, zwischen 15,24 und 17,15 cm, einer Wandstärke von 0,76 bis 2,5 mm und mit einen mit dem Pumpenkopf für dessen Drehung verbundenen Antrieb mit einer Geschwindigkeit im Bereiche von 200-600 1/min. Trotz der Vorteile der großen Effizienz und der kleinen Größe und des geringen Gewichtes der Dialysatlösungspumpe gemäß US No. 720 672 hat es sich herausgestellt, daß die Ausflußrate der Dialysatlösung mittels dieser bekannten Pumpe drastisch mit den Änderungen des negativen Drucks in der Dialysatlösung variiert. Eine derartige Flußratencharakteristik stellt einen schweren Nachteil für die bekannte Pumpe dar, wenn sie dafür verwendet wird, Dialysatlösung in das Hemodialysesystem zu pumpen. Dies deshalb, weil es im allgemeinen notwendig ist, das Niveau des negativen Drucks der Dialysatlösung welche durch den Dialysator gepumpt wird, von Patient zu Patient und für einen bestimmten Patienten von Dialyse zu Dialyse abhängig von der Höhe der notwendigen Wasserentfernung mittels Ultrafiltration bei einer speziellen Dialysebehandlung zu variieren. So dient z.B. die Erhöhung des negativen Drucks der Dialysatlösung für die Dialyse zur korrespondierenden Erhöhung des Druckgradienten und damit der Antriebskraft für den Massentransport von Wasser aus dem Blut durch die Massenübertragungsfläche des Dialysators zur Dialysatlösung, welche in indirekter Massenübertragungs-Dialysebeziehung zum Blut strömt, sodaß die vollständige Entfernung von Wasser aus dem Blut im Wege der Dialysebehandlung sichergestellt wird. Die oben beschriebene bekannte Pumpe gemäß US-No. 720 672 bewirkt eine Erhöhung des negativen Drucks mit drastisch reduziertem Ausfluß an Dialysatlösung aus der Pumpe. Die daraus resultierende Verminderung der Dialysatlösungsflußrate im Dialysatlösungskreislauf des Hemodialysesystems bewirkt eine Beeinträchtigung der Gesamteffektivität der Dialysebehandlung und erfordert eine deutlich größere Behandlungszeit, um die erforderliche Entfernung von Verunreinigungen aus dem Blut, welches dialysiert werden soll, zu erzielen.

Konfrontiert mit dem Problem der in einem sehr weiten Bereich variierenden Flußrate der Dialysatlösung von einer einzigen perstistaltischen Pumpe, welche den negativen Druck in der Dialysatlösung ändert, schlägt der Stand der Technik verschiedene Anordnungen vor, bei welchen zwei peristaltische Pumpem im Dialysatlösungskreislauf in Serie angeordnet sind, um einen relativ konstanten Fluß anstelle von Änderungen des negativen Drucks in der Dialysatlösung zu gewährleisten. In einer derartigen Anordnung wird eine erste peristaltische Pumpe stromauf des Dialysators angeordnet mit einer Durchfluß-Drosselvorrichtung zwischen der ersten peristaltischen Pumpe und dem Dialysator zur Erzeugung eines negativen Drucks in der durchfließenden Dialysatlösung. Eine zweite peristaltische Pumpe ist stromab des Dialysators angeordnet. Die Funktion der zweiten Pumpe liegt in der Angleichung der Änderung in der Dialysatlösungs-Flußrate von der ersten Pumpe mit den Änderungen infolge des negativen Drucks, d.h., in der Glättung der Ausflußrate von der ersten Pumpe, um eine im wesentlichen konstante Flußrate im Dialysat-Lösungsflußkreis aufrecht zu erhalten, statt des Wechsels infolge des negativen Drucks. Obwohl die Doppelanordnung von peristaltischen Pumpen das Problem der Änderungen in der Dialysatlösungs-Flußrate infolge des Wechsels des negativen Drucks in der Dialysatlösung bewältigt, bedeutet eine derartige Anordnung zusätzliche Unkosten und bringt die Komplexität einer zweiten peristaltischen Pumpe im Vergleich mit einem Hemodialyse-

0004600

system, welches nur eine einzige peristaltische Pumpe verwendet, mit sich.

Es ist Aufgabe der Erfindung, eine verbesserte peristaltische Pumpvorrichtung zum Pumpen der Dialysatlösung durch einen Dialysatlösungs-Kreislauf in einem Hämodialysesystem anzugeben.

Es ist eine weitere Aufgabe der Erfindung, eine einzige peristaltische Pumpe anzugeben, die geeignet ist, einen im wesentlichen konstanten Ausfluß von Dialysatlösung trotz der Änderungen des negativen Drucks der zu pumpenden Dialysatlösung vorzusehen.

Die Erfindung bezieht sich im allgemeinen auf peristaltische Pumpen zum Pumpen einer Dialysatlösung durch einen Dialysatlösungs-Kreislauf in einem Hämodialysesystem. Im besonderen bezieht sich die Erfindung auf einen Hämodialyse-Apparat zur Behandlung von Blut, um daraus die Verunreinigungen der Abfallstoffe zu entfernen. Die Vorrichtung umfaßt einen Dialysator, durch den das die Abfallstoffverunreinigungen enthaltende Blut und eine Dialysatlösung in indirekter Massenübertragungsdialysierungsbeziehung geleitet werden, um die Abfallverunreinigungen vom Blut auf die Dialysatlösung zu übertragen. Weiters ist eine Vorrichtung zur Übertragung des Abfallverunreinigungen enthaltenden Blutes vom Patienten zum Dialysator und zur Zurückführung des von Abfallverunreinigungen gereinigten Blutes zu dem Patienten vorgesehen. Ebenso ist eine Vorrichtung zur Übertragung der Dialysatlösung von einer Dialysatlösungsquelle zum Dialysator vorgesehen, zusammen mit einer Einrichtung zum Ablassen der mit Abfallverunreinigungen angereicherten Dialysatlösung aus dem Dialysator, wobei ein Dialysatlösungskreislauf gebildet wird, der einen flexiblen elastischen Schlauchpumpemabschnitt umfaßt, durch welchen die Dialysatlösung gepumpt wird. Eine peristaltische Pumpe mit einem drehbaren Pumpenkopf ist vorgesehen, dessen Basis zur Drehung um eine fixe Achse angeordnet ist und mehrere über den Umfang verteil-

0004600

te Rollen trägt, welche um entsprechende zur Achse der Basis parallele Achsen voneinander unabhängig drehbar sind.

Weiters ist eine Einrichtung zur Verankerung der Endabschnitte des flexiblen elastischen Schlauchpumpenabschnittes derart vorgesehen, daß sich der Schlauch gespannt um den Pumpenkopf erstreckt und mit zumindest zwei der am Umfang verteilten Rollen gleichzeitig im Eingriff steht und zusammengepreßt wird, wobei die Rollen zur Längsbewegung der Druckpunkte entlang des Schlauches während der Rotation des Pumpenkopfes befestigt sind, um die Dialysatlösung durch den Schlauch weiter zu bewegen.

Gemäß einem speziellen Ausführungsbeispiel der Erfindung umfaßt die peristaltische Pumpe vier auf der Basis befestigte Rollen, deren jede eine im wesentlichen konvexe Profilfläche aufweist, um den Schlauch an den Druckpunkten von den Rollen teilweise zu umschließen, mit einem maximalen Durchmesser zwischen 6,35 und 19 mm, wobei jede Rolle von der benachbarten bezüglich des Umfanges um 90° versetzt ist, wobei der radiale Abstand zwischen Rollenachse und der feststehenden Achse der Basis zwischen 12,7 und 38 mm beträgt. Der flexible elastische Schlauchpumpenabschnitt weist eine Länge, gemessen längs des Schlauches zwischen dessen Befestigungssegmenten, zwischen 17,15 und 28 cm und eine Wandstärke von 1,7-3,2 mm und einen inneren Durchmesser von 4,5-9 mm auf. Mit dem Pumpenkopf sind für dessen Drehung Antriebsmittel verbunden, welche diesen mit einer Geschwindigkeit im Bereich zwischen 50 und 400 1/min drehen.

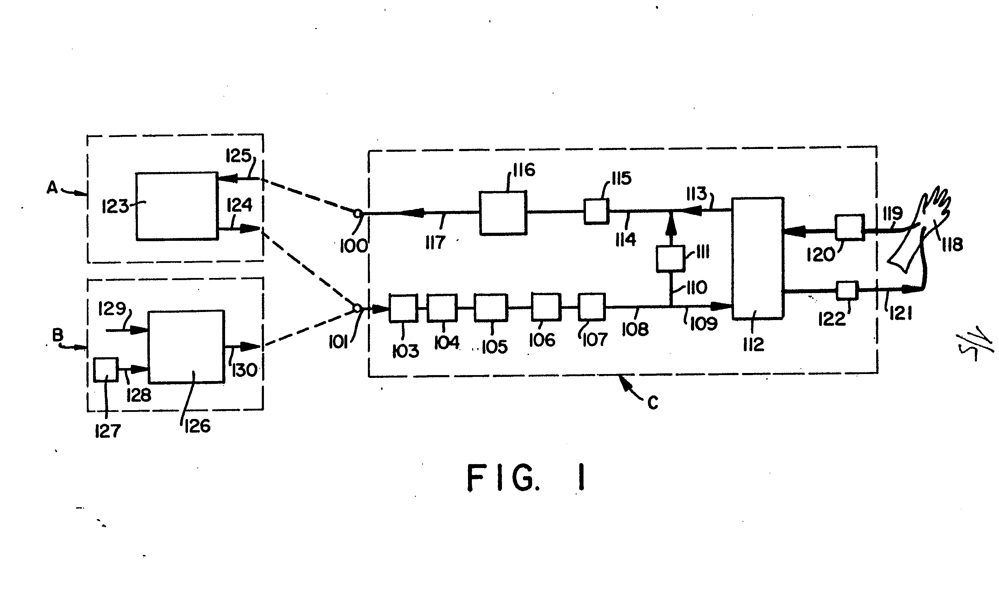

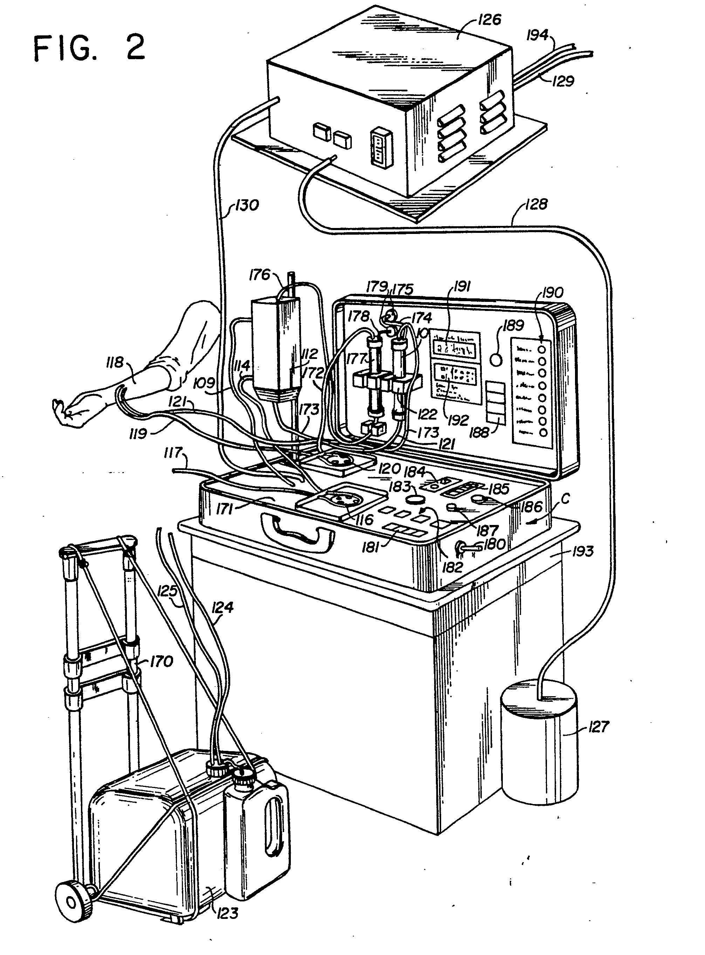

Nachstehend wird die Erfindung an Ausführungsbeispielen anhand der Zeichnungen näher erläutert. Es zeigen: Fig. 1 schematisch ein Blockdiagramm eines Hemodialysesystems, wie es z.B. in geeigneter Weise in Verbindung mit der vorliegenden Erfindung verwendet werden kann; Fig. 2 eine perspektivische Ansicht eines Hemodialysesystems, korrespon-

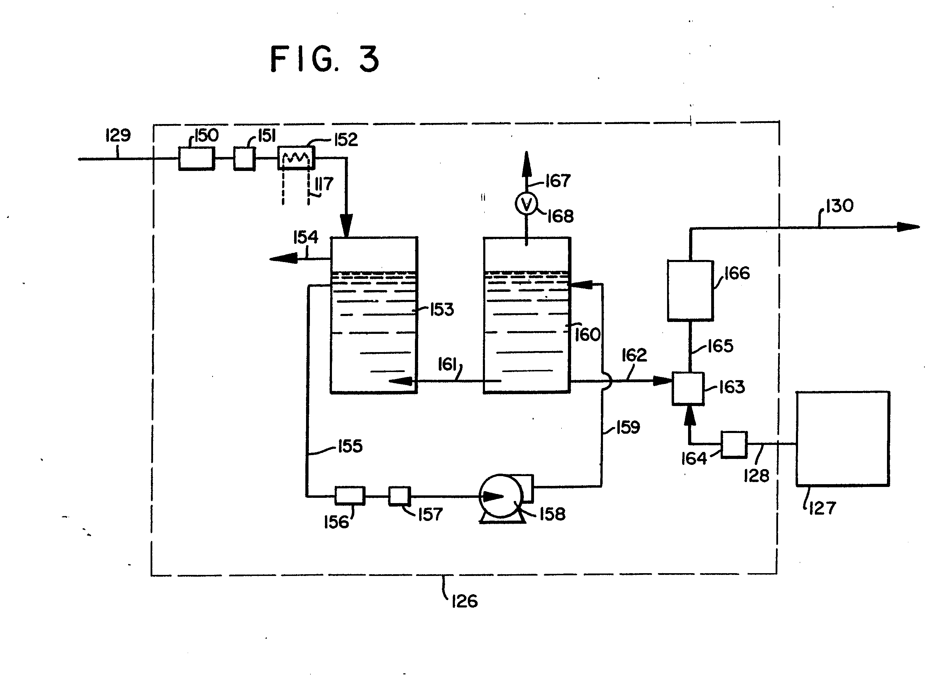

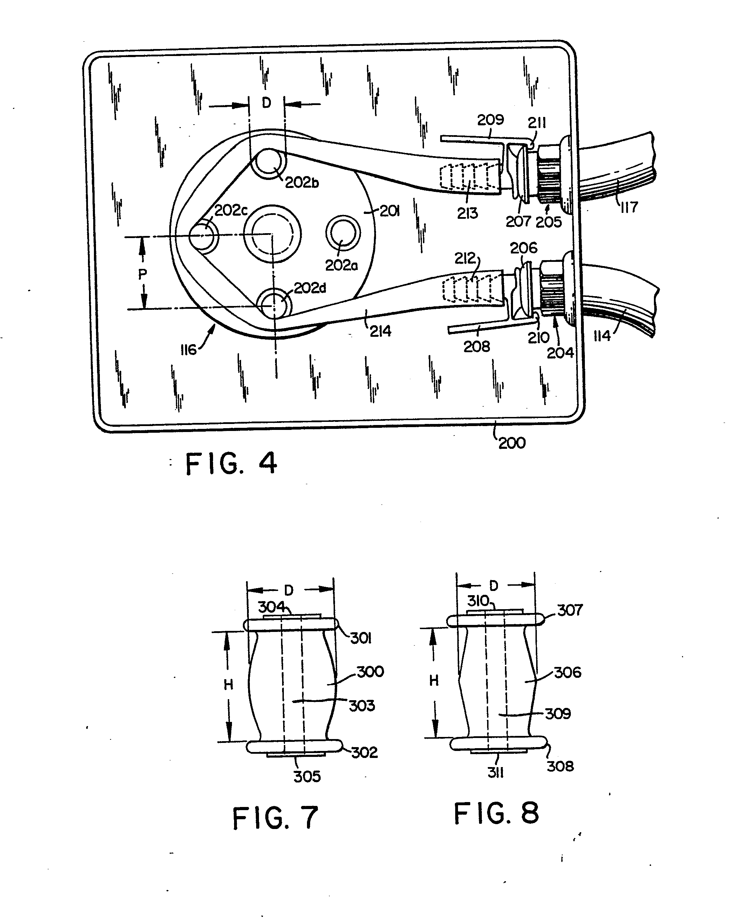

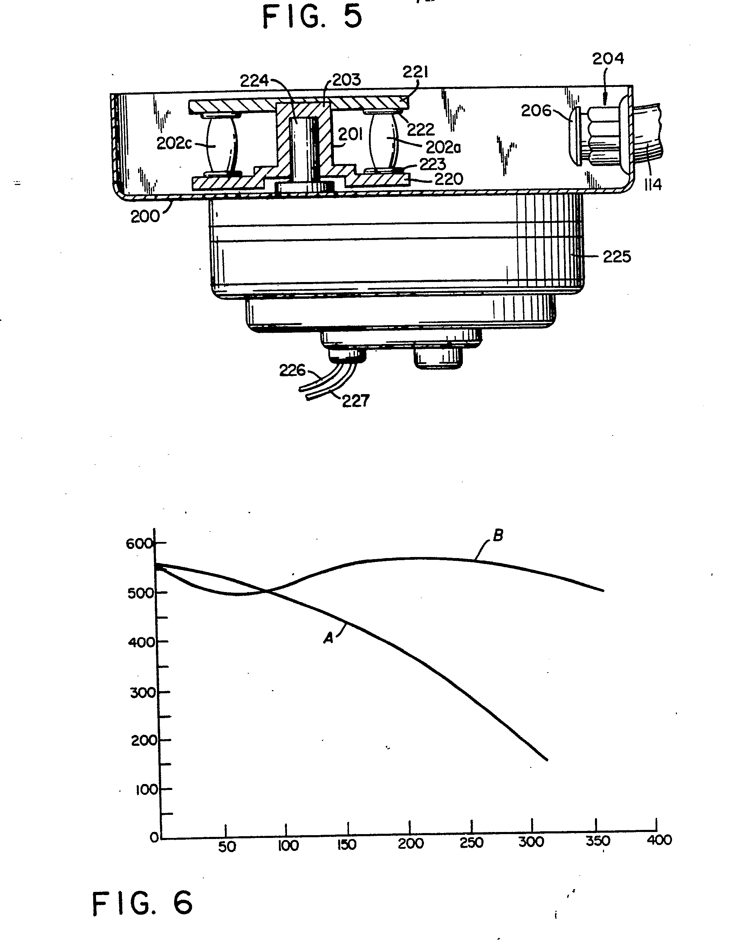

dierend zum Blockdiagramm von Fig. 1; Fig. 3 ein schematisches Diagramm eines Bemessungssystems, geeignet für die Verwendung in den Systemen gemäß Fig. 1 und 2; Fig. 4 eine Draufsicht auf eine Dialysatlösungspumpe gemäß der vorliegenden Erfindung, wie sie in geeigneter Weise im Hemodialysesystem gemäß Fig. 2 verwendet werden kann; Fig. 5 einen Aufriß der Dialysatlösungspumpe gemäß Fig. 4 im Schnitt zusammen mit einer angebauten Antriebsvorrichtung; Fig. 6 einen Graph der Dialysatflußrate in ml/min aufgetragen als Funktion des negativen Drucks der Dialysatlösung in mm Hg, der Leistungskurven für die Dialysatlösungspumpen der Fig. 4 und 5 und einer Dialysatlösungspumpe des Standes der Technik zeigt; Fig. 7 im Aufriß eine Rolle, wie sie für eine Dialysatlösungspumpe der Fig. 4 und 5 geeignet ist; und Fig. 8 einen Aufriß eines anderen Ausführungsbeispiels einer Rolle geeignet zur Verwendung in einer Dialysatlösungspumpe gemäß Fig. 4 und 5.

Die Fig. 1 zeigt ein schematisches Blockdiagramm eines Hemodialysesystemes, geeignet für die Verwendung in Verbindung mit der vorliegenden Erfindung. In diesem System ist der Patient oder Hemodialysegegenstand 118 angeschlossen, in einem geschlossenen Blutkreislauf, welcher von Blutflußleitungs-Abschnitten 119 und 121 mit einem Dialysator 112 gebildet wird, der von irgendeiner geeigneten Type sein kann, wie z.B. eine Type mit parallelen Platten oder eine Type mit einem hohlen Faserbündel. Das mit Abfallverunreinigungen versehene Blut wird vom Patienten mittels einer artheriellen Fistel, einer Kanüle oder einer Abzweigung (nicht gezeigt) abgezogen und vom Leitungsabschnitt 119 zu einer peristaltischen Blutpumpe 120 transportiert, welche stromauf des Dialysators 112 angeordnet ist. Durch die Blutpumpe 120 wird das Abfallverunreinigungen enthaltende abgezogene Blut peristaltisch gepumpt, um das Blut durch den oben beschriebenen Blutkreislauf vorzuschieben. Die Blutpumpe kann in geeigneter Weise eine der in US-PS 720 672, angemeldet am 7.9.1976 auf den Namen "J.T.Hutchisson" geoffenbarten Pumpe sein. Die Geschwindigkeit der peristaltischen Blutpumpe kann in geeigneter Weise durch eine Motorgeschwindigkeitssteuerung, die mit der Pumpe gekuppelt ist, gesteuert werden, um die notwendige Flußrate für Blut durch den Blutkreislauf z.B. 200 ml/min zu erhalten. Nachdem es durch die peristaltische Blutpumpe 120 gepumpt wurde, wird das die Abfallverunreinigungen enthaltende Blut über die Leitung 119 durch den Dialysator 112 in indirekter Massenübertragungs-Dialysebeziehung mit der Dialyselösung, die den Dialysator durch die Leitung 109 betritt, geleitet. Wie bereits angeführt kann der Dialysator in geeigneter Weise von der Parallelfluß-Hohlfasertype sein und ein gebündeltes Feld von hohlen Fasern umfassen, durch welche das die Abfallverunreinigungen enthaltende Blut im Gegenfluß zur Dialysatlösung geleitet wird, welche durch das gebündelte Feld entlang der äußeren Fläche der hohlen Fasern fließt. Das von Abfallverunreinigungen gesäuberte Blut wird vom Dialysator an den Patienten über die Blutrückflußleitung 121 zurückgeleitet. Die Blutrückflußleitung hat einen Luftblasendetektor 122 angeschlossen, der die Anwesenheit von irgendwelchen Gasblasen in dem an den Patienten rückgeführten Blut anzeigt. Der Detektor 122 kann in geeigneter Weise mit einer visuellen Anzeige oder mit einer hörbaren Alarmvorrichtung verbunden sein, um den Patienten auf die Anwesenheit von Gasblasen im zurückgeführten Blutstrom aufmerksam zu machen. Zusätzlich kann der Detektor operativ mit einer Klemmvorrichtung verbunden sein, welche die Blutrückführleitung 121 abklemmt, wenn Luft im Blut vom Detektor 122 entdeckt wird, sodaß die teilweise geklemmte Blutrückführleitung verhindert, daß irgendein Gas mit dem Blut in den Körper des Patienten zurückgeführt wird.

Wie aus Fig. 1 zu ersehen, ist das Hemodialysesystem in 3 getrennte Teile geteilt, deren jeder durch eine strichlierte Linie umschlossen ist und ein getrenntes Modul des gesamten Hemodialysesystems darstellt. Der Abschnitt C der Fig. 1 repräsentiert das Hemodialysemodul des gesamten Hemodialysesystems. Die Abschnitte A und B repräsentieren alternative Quellen für Dialysatlösung für das Hemodialysemodul C. Der Abschnitt A repräsentiert ein Dialysatlösungs-
füll- und Wiedergewinnungsmodul und umfaßt einen Dialysatlösungs-Speisebehälter 123, der mit Auslaß- und Rückführleitungen 124 und 125 versehen ist. Wie aus der Zeichnung
zu ersehen, können die Einlaß- und Rückführleitungen des
Füll- und Wiedergewinnungsmoduls mit dem Dialysatlösungs-
kreislauf im Hemodialysemodul C mittels der Verbindungen
100 und 101 verbunden werden. In diesem Fall wird ein
geschlossener Kreislauf für das Dialysatlösungsmittel
gebildet, wobei die Dialysatlösung vom Behälter 123 in die
Leitung 124, in die Leitung 102 eingeführt wird, um eventuell durch den Dialysator 112 zu fließen und wobei die mit
Abfallverunreinigungen angereicherte Dialysatlösung vom
Hemodialysemodul C in die Leitung 117, die mittels Verbinder 100 mit der Leitung 125 verbunden ist, ausgegeben wird,
um die Dialysatlösung in den Speisebehälter 123 zurückzuführen. Alternativ dazu kann das Bemessungsmodul B mit dem
Hemodialysemodul C verbunden werden, um einen offenen Kreislauf mit einem einfachen Durchgang für die Dialysatlösung
vorzusehen. Das Bemessungsmodul B umfaßt ein Bemessungssystem 126, welches einen kontinuierlichen Wasserzufluß in der
Leitung 129 von einer geeigneten Quelle erhält. In dem
Bemessungssystem 126 wird Wasser von der Leitung 129 und
ein Dialysatkonzentrat in die Leitung 128 vom Dialysatkonzentrat-Speisebehälter 127 eingeführt und in einem vorbestimmten Verhältnis miteinander gemischt, um eine Dialysatlösung für die Hemodialyse zu bilden, welche vom Bemessungssystem über die Leitung 130 ausgegeben wird.

Die Leitung 130 kann in geeigneter Weise mit der Leitung
102 des Hemodialysemoduls C mittels Verbinder 101 gekuppelt
sein. In der Betriebsweise mit offenem Kreislauf und Einfachdurchgang ist der Endabschnitt der Ausflußleitung 117
für Dialysatlösung in geeigneter Weise mit einem Kanal
oder einer anderen Abfallbeseitigungsvorrichtung verbunden
durch die getrennte Verwendung von Speisemoduls für Dialysatlösung für den offenen Kreislauf und für den geschlos-

0004600

senen Kreislauf ergibt sich eine große Vielseitigkeit für die Verwender von Hemodialysesystemen, insbesondere wenn die einzelnen Moduls A, B und C für leichte Transportierbarkeit ausgelegt sind, insofern als das Bemessungsmodul B bei jeder geeigneten Wasserquelle verwendet werden kann, während das Zuführ- und Wiedergewinnungsmodul A in anderen Fällen zur Anwendung kommt, unabhängig davon, ob das Dialysatlösungs-Speisemodul A oder B verwendet wird, ist die Funktion des Dialysatlösungskreislauf-Abschnittes verbunden mit dem Hemodialysemodul C, die gleiche. Die Dialysatlösung tritt in das Hemodialysemodul C durch die Leitung 102 ein und wird durch eine Heizeinrichtung 103 geleitet, in der die Dialysatlösung wenn nötig, auf 36,67-37,78°C erwärmt wird. Diese Erhitzung wird ausgeführt, um eine geeignete Temperatur für die Dialysatlösung zu erhalten, um ein unnötiges Erwärmen oder Kühlen des Blutes durch Wärmeaustausch zwischen Dialysatlösung und Blut zu verhüten und um Hemolyse zu verhüten. Warme Dialysatlösung fließt von der Heizvorrichtung 103 zur Temperaturfühlvorrichtung 104 für die Dialysatlösung.

Die Temperaturfühlvorrichtung weist eine Einrichtung zur Fühlung der Temperatur der Dialysatlösung zusammen mit einer Einrichtung zur Umwandlung des Dialysatlösungs-Temperaturfühlens in ein übertragbares Signal auf. Das Temperaturfühlsignal wird über geeignete Übertragungsmittel an einen Temperatursteuerkreis übertragen, der das Temperaturfühlsignal mit einem eingestellten Wert vergleicht und ein resultierendes Steuersignal erzeugt, welches durch geeignete Steuersignalübertragungsmittel an die Heizvorrichtung 103 übertragen wird, um das erforderliche Temperaturniveau zur Einhaltung des eingestellten Wertes vorzusehen. Auf diese Weise ist die Aufheizung der Dialysatlösung durch die Heizvorrichtung in Abhängigkeit von der Temperaturfühlung in der Vorrichtung 104 eingestellt, um ein voreingestelltes Temperaturniveau für die Dialysatlösung aufrecht zu erhalten. Zusätzlich zur Steuerfunktion, bedient durch das Temperaturfühlsignal in der Temperaturfühlvorrichtung 104 kann das Fühlsignal auch zur Betätigung einer sichtbaren Temperaturanzeige oder eines hörbaren Alarms verwendet werden, wenn die Temperatur der Dialysatlösung zulässige Werte überschreitet.

Von der Temperaturfühlvorrichtung 104 wird die Dialysatlösung zur Leitfähigkeitsmeßvorrichtung 105 geleitet. In der Vorrichtung 105 ist eine Einrichtung zur Messung der elektrolytischen Leitfähigkeit der Dialysatlösung zusammen mit einer Einrichtung zur Umwandlung der elektrolytischen Leitfähigkeitsmessung der Dialysatlösung in ein übermittelbares Signal vorgesehen. Das übermittelbare Signal kann mittels geeigneter Signalübermittlungseinrichtungen zu einer geeigneten visuellen Anzeige übertragen werden, um die gemessene elektrolytische Leitfähigkeit der Dialysatlösung anzuzeigen. Das Anzeigen der Leitfähigkeit ist wünschenswert, um sicherzustellen, daß die Dialysatlösung das richtige Niveau an Salzhaltigkeit und elektrolytischem Charakteristikas aufweist, sodaß vitale Komponenten des Blutes nicht an die Dialysatlösung durch Ionendiffusion über die Massenübertragungsfläche im Dialysator verlorengehen.

Von der Leitfähigkeits-Meß-Vorrichtung 105 wird die Dialysatlösung durch die negative Druckregelungs/Flußsteuerungsvorrichtung 107 geleitet. Diese Vorrichtung weist eine Einrichtung zur Einstellung des negativen Drucks auf und besitzt eine Anzeigeeinrichtung für den negativen Druck.

Wie weiter oben aufgezeigt, wird der negative Druck auf der Seite der Dialysatlösung des Massenübertragungselementes im Dialysator angewendet, um die Entfernung von Wasser aus dem Blut mittels Ultrafiltration zu bewirken. Der negative Druck der Dialysatlösung kann in der Einrichtung 106 durch eine Einstellvorrichtung, wie z.B. ein elliptisches Flußventil oder ein Nadelventil, welches zur Regulierung des negativen Drucks und der Steuerung der Flußrate der Dialysatlösung durch den Kreislauf dient, eingestellt werden.

0004600

Stromab von der negativen Druckregelungs/Flußsteuerungsvor-richtung 106 ist eine negative Druckanzeigevorrichtung 107 angeordnet. Diese kann in geeigneter Weise einen Negativdrucksensor umfassen, der operativ mit einer visuellen Anzeige zum Anzeigen der Größe des negativen Drucks der Dialysatlösung verbunden ist. Der Negativdrucksensor kann auch mit der Negativ-Druckeinstellvorrichtung 106 operativ verbunden sein, wobei der negative Druck der Dialysatlösung auf einem vorbestimmten Niveau aufrechterhalten wird.

Die vom Negativdrucksensor 107 austretende Dialysatlösung wird in die Leitung 108 und zur Leitung 109 und von der Leitung 109 in den Dialysator 110 geleitet. Im Dialysator wird die Dialysatlösung in indirekter Massenübertragungs-Dialysierungsbeziehung mit dem Abfallverunreinigungen enthaltenden Blut geleitet, welches vom Blutkreislauf mit dem Blut Leitungen 119, 121 hier durchfließt. Aus einer derartigen Massenübertragung im Dialysator 112 ergibt sich, daß die Abfallverunreinigungen vom Blut auf die Dialysatlösung übertragen und die sich ergebende mit Abfallverunreinigungen angereicherte Dialysatlösung vom Dialysator 112 in die Leitung 113 abgeleitet wird. Von der Leitung 113 wird die Dialysatlösung in die Leitung 114 zur Blutleckanzeige geleitet. Diese Vorrichtung weist eine Einrichtung zur Auffindung von Blutleckmengen im Kreislauf der Dialysatlösung zusammen mit einer Einrichtung zur Umwandlung der festgestellten Blutleckmengen in ein übertragbares Signal auf. Dieses Signal wird durch geeignete Übertragungsmittel an eine Ausgabevorrichtung für die Blutleckmengen-Entdeckung übertragen, welche in geeigneter Weise eine sichtbare Anzeige oder eine hörbare Alarmvorrichtung umfaßt. Diese Blutleckentdeckungs-Vorrichtung ist vorgesehen, um sicherzustellen, daß nur eine direkte Massenübertragung - d.h. eine Übertragung mittels Diffusion und Osmose von Species über das Dialysations-Massenübertragungselement - stattfinden kann, ohne daß ein gegenseitiges Lecken der Flüssigkeiten in dem Dialysator auftritt. Nachdem die Dialysatlösung durch den Blutleckdetektor 115 geflossen ist, wird sie durch eine peristaltische Pumpe 116, welche die Dialysatlösung durch den Kreislauf der Dialysatlösung weitertransportiert, gepumpt und die sich ergebende gepumpte Dialysatlösung vom Hämodialysemodul C an der Leitung 117 abgegeben.

In dem oben beschriebenen Dialysatlösungs-Kreislaufabschnitt in Verbindung mit dem Hämodialysemodul C ist eine Verzweigungsleitung 110 mit einer Bypass-Regelung 111 angeordnet. Der Zweck für den Bypass-Regulator 111 in der Zweigleitung 110 ist die Ableitung der Dialysatlösung von der Leitung 108 in die Leitung 114, sodaß die Dialysatlösung am Dialysator 112 vorbeigeleitet wird. Der Bypass-Regulator 111 ist derart angeordnet und konstruiert, daß die Dialysatlösung durch die Leitung 110 abgeleitet und am Dialysator 112 vorbeigeleitet wird, wenn die Dialysatlösung die zur Dialyse geeigneten Charakteristika nicht aufweist. Für diesen Fall kann der Bypass-Regulator 111 operativ mit einem oder mehreren der Temperaturfühler 104, Leitfähigkeitsmesser 105, Negativdruck-Feststell-Vorrichtung 107 und Blutleck-Feststell-Vorrichtung 115 verbunden sein, sodaß die Dialysatlösung in den Bypass-Fluß strömt, wenn die festgestellten Charakteristika der Dialysatlösung erlaubte Grenzwerte überschreiten, oder wenn eine Blutleckage in die Dialysatlösung durch den Blutleck-Detektor 115 festgestellt wird.

Fig. 2 ist eine perspektivische Ansicht eines kompakten transportablen Hämodialyse-Systems, dessen allgemeiner Typ in Fig. 1 gezeigt ist. Entsprechende Systemelemente haben in Fig. 1 und 2 die gleichen Bezugszeichen.

Wie in Fig. 2 gezeigt, ist das Hämodialysemodul C in einem einstückigen kofferartigen Behälter enthalten. Der Behälter umfaßt einen oberen und unteren Abschnitt, die miteinander gelenkig verbunden sind und welche, wenn das Dialysemodul nicht verwendet wird, mittels geeigneter komplementärer Sperrglieder zusammengesteckt und zusammengehalten sind. Im

0004600

so zusammengeklappten Zustand ist der Hämodialysebehälter sehr kompakt und ist z.B. 53 cm lang, 30 cm breit und 15 cm hoch. In der Praxis kann der Behälter aus Leichtmetall, wie z.B. Aluminium gebildet sein, sodaß das Gewicht des gesamten Behälters wegen der Transportierbarkeit sehr gering gehalten wird, z.B. in der Größe von 12 kg.

Der Hämodialysemodul ist so ausgelegt, daß mit dem bekannten 120/220 V Wechselstrom betrieben werden kann, welcher über die Stromleitung 180 in den Unterteil des Moduls eingeleitet wird. Im unteren Abschnitt des Behälters ist unterhalb der sichtbaren Platte eine Gleichstromquelle angeordnet, um den für die verschiedenen Anzeigevorrichtungen und Anzeigen benötigten Strom zur Verfügung zu stellen.

Wie gezeigt, ist der Hämodialyse-Koffermodul C auf einem Ständer 193 angeordnet. Das Bemessungssystem 126 ist auf einem oberhalb des Hämodialyse-Koffermoduls angeordneten Brett und der Dialysatkonzentrat-Behälter 127 am Boden neben dem Ständer 193 angeordnet. Im Anschluß an das Hämodialysesystem ist die Dialysatlösungs-Füll- und Rückführvorrichtung angeordnet, die einen Behälter 123 für die Dialysatlösung, versehen mit Zuführeinlaßleitung und Rückführleitung 124 und 125 umfaßt. Der Behälter 123 ist zur leichteren Transportierbarkeit auf einer Transportvorrichtung 170 angeordnet.

Der Blutkreislauf des Systems der Fig. 2 umfaßt Leitungsabschnitte 119, 172, 176, 173 und 121, die aus irgendeinem bekannten transparenten Polyvinylchlorid, Polyurethan oder Siliconelastomer gebildet sein können. Das Abfallverunreinigungen enthaltende Blut wird vom Patienten mittels einer arteriovenösen Fistel abgezogen und in eine arterielle Zuführleitung 119 und zu einer peristaltischen Blutpumpe geleitet, welche in geeigneter Weise von jener Type sein kann, die in der US S.No. 720 672, angemeldet am 7.9.1976 auf den Namen "J.T.Hutchisson", geoffenbart und beansprucht ist und hiermit hierin der Sache nach inkorporiert ist. Das peristaltisch gepumpte Blut von der Blutpumpe 120 wird in die Leitung 172 zur arteriellen Tropfkammer 177 geleitet. Der Druck der arteriellen Tropfkammer wird mittels einer Druckanzeige-Verbindungsleitung 178 gemessen, welche die Tropfkammer 177 mit einer Druckanzeigevorrichtung 179 verbindet. Von der arteriellen Tropfkammer wird das Blut in die Leitung 176 und durch den Dialysator 112 geleitet. Der Dialysator 112 kann in geeigneter Weise von der Parallelfluß-Hohlfasertype sein, wie er vorhin beschrieben wurde und z.B. ca. 1,5 m$^2$ Massenübertragungs-Membranfläche für die Dialyse aufweisen. Im Dialysator diffundieren die Abfallveinigungsbestandteile des Blutes, wie z.B. Harnstoff, Harnsäure und Kreatinin vom Blut über die Membrane in die Dialysatlösung. Wasser wird aus dem Blut entfernt mittels Ultrafiltration, welche unter Verwendung eines negativen Drucks von bis zu 350 mm Hg auf der Dialysatlösungsseite der Membran und bis zu 500 mm Hg Druckdifferenz über die gesamte Membran ausgeführt wird. Üblicherweise werden 1-2 1 Wasser während der Dialysebehandlung entfernt.

Vom Dialysator 112, der an der Seite des Behälters mittels eines Ständers und mittels Klammern unterstützt angeordnet ist, welche mit der Seitenwand des unteren Abschnitts des Behälters verbunden sind, fließt das von Abfallverunreinigungen gesäuberte Blut in die Leitung 173 zur venösen Tropfkammer, an die eine Luftleckaufspürvorrichtung 122 angeschlossen ist, welch letztere dazu dient, die Anwesenheit von Gasblasen in dem Blut, welches die venöse Tropf-kammer durchfließt, festzustellen. Eine Druckanzeigever-bindungsleitung 174 ist mit der venösen Tropfkammer verbunden und kommuniziert mit der venösen Druckanzeigevorrichtung 175, welche den Druck in der venösen Tropfkammer anzeigt. Von der venösen Tropfkammer wird das von Verunreinigungen gesäuberte Blut an den Patienten über die Leitung 121 zurückgegeben.

Der Dialysatlösungs-Kreislauf für das System der Fig. 2 kann z.B. ein offener Kreislauf (d.h. Einmaldurchfluß) oder ein geschlossener Kreislauf sein, u.zw. abhängig davon, ob der Füll- und Rückführbehälter 123 oder das Bemessungssystem 126 als Quelle für die Dialysatlösung für das Hämodialysemodul dient. In Fig. 2 ist das Bemessungssystem 126 operativ verbunden mit dem Hemodialysemodul C gezeigt, sodaß das Hämodialysesystem für einen offenen Kreislauf und Einfachdurchfluß der Dialysatlösung ausgebildet ist. In der gezeigten offenen Kreislaufanordnung umfaßt der Dialysatlösungs-Kreislauf Leitungsabschnitte 130, 109, 114 und 117, welche ebenfalls von bekannter Art, z.B. aus Polyvinylchlorid, Polyurethan oder Silicongummi gebildet sein können.

Das Bemessungssystem 126 für den offenen Kreislauf der Dialysatlösung wird nachfolgend im größeren Detail beschrieben und ist in einem einstückigen Behälter wie gezeigt, vorgesehen. Das Bemessungssystem ist dafür ausgelegt, mit üblichem 120 oder 220 V Wechselstrom betrieben zu werden, wie er dem Bemessungssystem über die Stromleitung 194, die in den Behälter auf dessen Rückseite führt, zugeführt wird. Dem Bemessungssystem 126 wird Wasser über die Leitung 129, welche in geeigneter Weise mit einer Wasserquelle verbunden ist, wie z.B. ein Haushaltswasserhahn, zugeführt. Das Dialysatkonzentrat ist für das Bemessungssystem aus einem Behälter 127 vorgesehen, und fließt von diesem durch die Leitung 128 zum Bemessungssystem 126, um darin mit einer vorausbestimmten Menge Wasser aus der Leitung 194 vermischt zu werden, um die Dialysatlösung für die Hemodialyse zu bilden. Die Dialysatlösung wird vom Bemessungssystem 126 über die Leitung 130 ausgegeben. Von der Leitung 130 führt die Dialysatlösung durch verschiedene Fühl- und Steuermechanismen, wie vorhin beschrieben, in Zusammenhang mit der Fig. 1 und in Verbindung mit dem System der Fig. 2 mit der lösbaren Dialysatlösungs·Mannigfaltigkeit 171. Diese Dialysatlösungsanordnung kann in geeigneter Weise im allgemeinen ähnlich jener in der US-S.No. 720 673, angemeldet am 7.9.1976 im Namen von "J.T.Hutchisson", ausgebildet sein. Nach dem Durchgang der Dialysatlösung durch die verschiedenen Fühl- und Steuereinrichtungen, verbunden mit der Dialysatanordnung 171 wird die Dialysatlösung in die Leitung 109 zum Dialysator 112 für die Übertragung der Abfallverunreinigungen vom Blut auf die Dialysatlösung geleitet. Die mit Abfallverunreinigungen angereicherte Dialysatlösung wird vom Dialysator 112 in die Leitung 114 ausgegeben und zur peristaltischen Dialysatlösungspumpe 116, wie nachher eingehend beschrieben, geleitet. Die Dialysatlösung wird im Dialysatlösungs-Kreislauf durch die peristaltische Pumpe 116 weiterbefördert und die Dialysatlösung wird von der Pumpe vom System in die Leitung 117 ausgegeben. Wie schon früher beschrieben, kann die Leitung 117 in geeigneter Weise mit einem Abfluß oder einer anderen Abfallbeseitigungsvorrichtung für die mit Verunreinigungen angereicherte Dialysatlösung verbunden werden.

Bei der Betriebsart mit geschlossenem Kreislauf und Dialysatlösungs-Füll- und Rückführung, wobei das Bemessungssystem 126 nicht verwendet wird, wird die Dialysatlösung vom Behälter 123 in der Leitung 124 abgezogen und von der Leitung 124 in die Dialysatanordnung 171 geleitet, anstatt des vorhin beschriebenen Dialysatlösungs-Einlaßleitung 130 in Verbindung mit dem Bemessungssystem 126. Zur Rückführung der Dialysatlösung vom Dialysat-Lösungskreislauf zum Behälter 123 ist die Ausflußleitung 117 der Dialysatlösungspumpe 116 über geeignete Mittel (nicht gezeigt) mit der Rückflußlinie 125 verbunden, welche selbst mit dem Dialysatlösungs-Behälter 123 verbunden ist.

Auf diese Weise wird die Dialysatlösung vom Behälter 123 in die Leitung 124 abgezogen und fließt durch den Dialysatlösungs-Kreislauf, verbunden mit dem Hemodialysemodul C in der gleichen Weise, wie vorhin beschrieben, in Verbindung mit der Verwendung des Bemessungssystems 126, aber mit der Rückführung der mit Verunreinigungen angereicherten Dialysatlösung von der Ausgabeleitung 117 durch die Rückflußleitung 125 zum Dialysatlösungsbehälter 123.

Auf der Vorderplatte des oberen Abschnittes des Kofferbehälters sind mehrere Sicherheitskontrolleuchten 190 vorgesehen, um den Zustand des Systems jederzeit anzuzeigen. Das oberste Licht in der Reihe ist grün und leuchtet auf, wenn alle Komponenten des Hämodialysesystems innerhalb erlaubter Grenzen arbeiten. Die verbleibenden Lichter der Reihe sind rot. Das unterste Licht der Reihe leuchtet auf, wenn der Dialysatlösungskreislauf des Hämodialysemoduls im Bypass arbeitet, wie früher beschrieben in Verbindung mit Fig. 1, weil eine oder mehrere der angezeigten Charakteristika der Dialysatlösung die zulässigen Arbeitsbedingungen überschreiten. Die verbleibenden Leuchten sind Warnleuchten, welche aufleuchten, wenn eine angezeigte Charakteristik der Dialysatlösung die zulässigen Grenzen überschreitet. Die Kontrolleuchten sind der Reihe nach vorgesehen für die Temperatur der Dialysatlösung, die Leitfähigkeit der Dialysatlösung, den venösen Druck, wie er durch die Druckanzeigevorrichtung 175 angezeigt wird, für den arteriellen Druck, angezeigt durch die Druckanzeigevorrichtung 179, für den Negativdruck der Dialysatlösung, für die Blutleckage in die Dialysatlösung und Luftleckage oder die Anwesenheit von Gasblasen im Blutkreislauf. Die Anzeigevorrichtungen im Hemodialysemodul sind operativ so angeordnet, daß sie die akustische Alarmvorrichtung 189 betätigen können, wenn einer oder mehrere der angezeigten Parameter die zulässigen Arbeitsgrenzen überschreiten.

Ebenfalls auf der Vorderplatte des oberen Abschnittes des Kofferbehälters ist eine Anzahl von Schaltern 188 angeordnet. Der obere Schalter ist eine akustische Alarmübersteuerung, welche, wenn der zugehörige Druckknopf eingedrückt ist, die akustische Alarmvorrichtung 189 zum Schweigen bringt. Der nächste Schalter ist eine Alarmübersteuerung, welche, wenn eingedrückt, die gesetzten Punkte für Blutleckage und Luftleckagealarm übersteuert. Der nächste Schalter ist in geeigneter Weise beleuchtet, um die normale Funktion des Hemodialysesystems anzuzeigen. Der unterste Schalter hat zwei Stellungen, welche die eingestellten Werte der Temperatur für die Dialysatlösungs-Heizung steuern. Die zwei Stellungen des Schalters entsprechen einer Dialysattemperatur von 36,67° und 37,78°C, welche die Heizrate der Dialysatlösung durch die Heizvorrichtung in der Dialysatanordnung zur Steuerung der Dialysatlösungstemperatur auf einem ausgewählten Niveau steuern. Unter normalen Arbeitsbedingungen wird die Stellung von 36,37°C gewählt. Bei niedrigen Außentemperaturen wird der eingestellte Wert von 37,78°C gewählt, um das Blut, das in indirekter Wärmeaustauschbeziehung mit der Dialysatlösung im Dialysator strömt auf etwa normaler Körpertemperatur zu halten.

Benachbart der akustischen Alarmvorrichtung 189 und den Sicherheitskontrollschaltern 188 sind auf der Frontplatte des oberen Abschnitts des Kofferbehälters numerische Anzeigevorrichtungen 191 und 192 angeordnet. Die obere numerische Anzeige 191 sieht eine kontinuierliche numerische Anzeige des venösen Druckes, wie er von der venösen Druckanzeigevorrichtung 155 angezeigt wird, vor. Die untere Anzeige 192 ist verbunden mit der Fühlvorrichtung für die Dialysatlösungstemperatur, für die Dialysatleitfähigkeit, für den Negativdruck der Dialysatlösung und für den arteriellen Blutdruck und die speziell angezeigten Parameter werden jeweils mittels des Anzeigeschalters 181, der an der Frontplatte des unteren Abschnitts des Kofferbehälters angeordnet ist, ausgewählt. In der Anordnung von Schaltern 181 sind einzelne Schalter vorgesehen, für die Auswahl jedes einzelnen der vorhin genannten Anzeigeparamter, sodaß durch Druck eines geeigneten Knopfes in Verbindung mit den Schaltern 181 die Übertragung eines Signals von der Fühlvorrichtung zugeordnet dem speziellen Parameter, auf die sichtbare Anzeige 192 hervorruft, sodaß augenblicklich der numerische Wert des aufgezeigten Parameters auf der Anzeige 192 angezeigt wird. Direkt oberhalb der Anordnung von Anzeigeschaltern 181 ist eine Gruppe von vier Schaltern 182 angeordnet. Diese Schalter schließen einen Schalter zur Betätigung der Dialysatlösungspumpe 116 ein, einen Schalter zur Lösung der eingestellten Grenzen der angezeigten Parameter, verwendet, um eine Betätigung der verschiedenen akustischen und visuellen Alarmvorrichtungen während des Anlaufens des Hemodialysesystems zu verhindern, einen Schalter, der, wenn er eingedrückt wird, die eingestellten Werte der verschiedenen angezeigten und gesteuerten Arbeitsparameter für die normale Dialysedurchführung einstellt und einen Schalter, der die verschiedenen Anzeige- und Steuervorrichtungen, während der Reinigung des Systems stillegt.Oberhalb der vorhin genannten Reihe von Schaltern ist auf der Frontplatte des unteren Abschnittes des Kofferbehälters eine Kontroll- und Einstellvorrichtung 183 für die Geschwindigkeit der Blutpumpe angeordnet, welche mit einer veränderlichen Motorgeschwindigkeitssteuerung verbunden ist, durch welche die Antriebsmittel mit der Blutpumpenkopfanordnung 120 für deren Drehung verbunden sind, u.zw. bei einer Geschwindigkeit z.B. im Bereich zwischen 50 und 100 1/min, ferner eine Sicherung 187, eine Steuerung 184 für die Einstellung des Leitfähigkeitsmessers, welche verwendet wird, um die am numerischen Display 192 angezeigte Leitfähigkeit einzustellen, zur Kalibrierung des Leitfähigkeits-Anzeigesystems im Bezug auf eine Lösung mit bekannter elektrolytischer Leitfähigkeit, einen Wechselstromzähler 185 zur Anzeige der Menge an Strom, welche das Hemodialysemodul von der Kraftleitung 180 erhalten hat, und eine Kalibriervorrichtung 186 für die Empfindlichkeit des Blutleckdetektors, welche dazu verwendet werden kann, das Anzeigeniveau, bei welchem der Blutleckdetektor die Anwesenheit vom Blut in der Dialysatlösung, welche durch den Abschnitt des Dialysatlösungs-Kreislaufes stromab des Dialysators 112 fließt, einzustellen.

Fig. 3 ist ein schematisches Blockdiagramm für ein Bemessungssystem 126, geeignet für die Verwendung in einem Hemodialysesystem der Fig. 1 und 2. Bei diesem wird Wasser von einer geeigneten Quelle, z.B. einem Wasserhahn, in das Bemessungssystem 126 über die Leitung 129 eingeführt. Vor der Einleitung des Wassers in das Bemessungssystem kann das Wasser filtriert, deionisiert oder einem umgekehrten Osmosevorgang unterworfen werden, um die unerwünschten Bestandteile aus dem Wasser zu entfernen. Geeignete Vorrichtungen für die Durchführung der angeführten Wasserbehandlungen sind dem Fachmann gut bekannt und stellen keinen Teil der vorliegenden Erfindung dar. Das in das Bemessungssystem 126 durch die Leitung 129 fließende Wasser fließt zuerst durch einen Druckschalter 150, der geeignet ist, den Wasserfluß durch die Leitung 129 zu unterbrechen, wenn der Wasserdruck ein vorausbestimmtes Niveau überschreitet, oder unter ein vorausbestimmtes Niveau fällt. Der Druckschalter kann von einer bekannten Type sein und die eingestellten oberen und unteren Grenzen für den Druckschalter sind z.B. 6,9 bis 1,3 bar. Diese eingestellten Grenzpunkte für die Druckabschaltung werden angewendet, um die Möglichkeit der Zerstörung einer Systemkomponente durch zu hohen Druck, oder durch zu niedrigen Druck zu vermeiden. Vom Druckschalter 150 strömt das Wasser in der Leitung 129 durch eine Flußsteuerung 151, welche eine konstante Durchflußrate für das Wasser in der Leitung 129 aufrechterhält. Die Flußsteuerung kann in geeigneter Weise von jener Type sein, welche dazu entworfen ist, eine konstante Druckdifferenz über ein integrales, von Hand betätigtes Flußregelventil aufrechtzuerhalten. In der Flußsteuerung ist ein inneres Membran-betätigtes Steuerventil eingestellt, das durch die Kraft, ausgeübt durch den Druck der einströmenden Flüssigkeit auf einer Seite der Membran und entgegen dem Druck der ausströmenden Flüssigkeit und einer Ventilvorspanneinrichtung auf der anderen Seite der Membran. Auf diese Weise stören Änderungen des stromaufwärts oder stromabwärts gelegenen Druckniveaus das Kräftegleichgewicht auf der Membran und veranlassen dadurch das Steuerventil zu öffnen oder zu schließen, wie es notwendig ist, um eine bestimmte Druckdifferenz über das Handflußregelventil aufrechtzuerhalten. Im Betrieb ist das Handflußregelventil eingestellt, um eine Flußrate von 500 bis 600 ml/min in die Leitung 129 abzugeben. Eine für diesen Zweck geeigneten Flußsteuerung ist im "Brooks Model Serie 8800" hergestellt von "Brooks Instrument Division of Emerson Electric Company, Hatfield, Pennsylvania" gegeben.

Danach fließt das Wasser in die Leitung 129 zur Heizeinrichtung 152, in der die Wassertemperatur auf ca. 32,2°C

angehoben wird. Die Heizeinrichtung kann für diesen Zweck jede geeignete bekannte Type sein. Wie schon früher angeführt, muß die Dialysatlösung, welche durch den Dialysator geleitet wird, ca. bei der normalen Körpertemperatur (37°C) liegen, um ein unerwünschtes Aufheizen oder Kühlen des Blutes infolge der Wärmeübertragung im Dialysator auszuschließen. Demgemäß wird das Wasser in der Leitung 129 auf ein Niveau erhitzt, welches der für die Dialysatlösung endgültig gewünschten Temperatur sehr nahe kommt, aber etwas unterhalb liegt, wobei die Dialysatlösung aus der Mischung von Wasser und Dialysatkonzentrat gemischt wird. Auf diese Weise muß die Dialysatlösung, welche vom Bemessungssystem ausgegeben wird, nur geringfügig weiter im Dialysemoldul C der Fig. 1 und 2 aufgeheizt werden. Zu diesem Zweck, d.h. für die notwendige geringfügige Aufheizung, kann die Dialysatlösungsheizung im Hemodialysemodul C relativ klein sein, wie es für die Kompaktheit und Transportierbarkeit des Hemodialysemodul-Kofferbehälters (Fig. 2) erwünscht ist. Um die Heizanforderungen für die Heizung 152 zu minimieren, kann diese Heizvorrichtung in geeigneter Weise einen Wärmetauscher zur Durchleitung von Wasser von der Leitung 129 im indirekten Wärmeaustausch mit der vom Dialysator abgegebenen Abfalldialysatlösung aufweisen, welch letztere durch den Wärmetauscher in der Leitung 117 zur Rückgewinnung der Wärme aus der Abfalldialysatlösung fließt. Ein derartiger Wärmetauscher minimiert die notwendige aufzubringende Wärme aus dem Netz, um die Temperatur des einfließenden Wassers auf das gewünschte Niveau anzuheben und läßt auf diese Weise die Verwendung einer relativ kleinen Heizvorrichtung für das Bemessungssystem zu.

Das das Bemessungssystem durch die Leitung 129 betretende Wasser enthält charakteristischerweise mitgeführte und gelöste Gase, wie sie z.B. von der Entlüftung und der Mitführung von Gas in Wasserzuführrohren und Haushalts-Fittings stammen. Insofern als die Temperatur des Wassers in der Leitung 129 deutlich erhöht wird, z.B. von einer Temperatur von 1,7°C auf ca. 32°C, werden die in der Wassereintrittsleitung 129 gelösten Gase durch die erhöhte Temperatur der Heizeinrichtung 152 abgeschieden, als Ergebnis der Tatsache, daß die Gaslöslichkeit in Flüssigkeiten im allgemeinen mit steigender Temperatur abnimmt. Daher enthält das von der Heizvorrichtung 152 mit erhöhter Temperatur abgegebene Wasser einen deutlichen Anteil von abgeschiedenen Gasen. Im allgemeinen ist die Anwesenheit von Wasser in der Dialysatlösung, die durch den Dialysator geführt wird, für die Sicherheit und die Effizienz der Hämolysebehandlung äußerst schädlich. Daher ist das Bemessungssystem 126 mit einer Entlüftung zur Entfernung lösbarer Gase aus dem erhitzten Wasser vor den Durchgang durch die Bemessungseinrichtung versehen. Von der Heizvorrichtung 152 wird das erhitzte Wasser, welches abgeschiedene Gase enthält, in die Leitung ausgegeben, bei einer Flußrate von z.B. 550 ml/min und einer Temperatur von 29,4 bis 35°C und einem Druck von 1,3-6,9 bar und zum ersten Speichertank 153 geleitet. Der erste Speichertank 153 hat ein Volumen von 150-400 ml und kann in geeigneter Weise ein zylindrisches Gefäß aufweisen, welches einen Durchmesser von 5 cm und eine Höhe von 18 cm aufweist. Der erste Speicherbehälter 153 ist mit einer Entlüftungsvorrichtung , wie z.B. einem Entlüftungsrohr 154 versehen, um die abgeschiedenen Gase, welche während des Heizens in der Heizvorrichtung 152 vom Wasser abgeschieden wurden, vom erwärmten Wasser ablösen zu lassen, um teilweise entlüftetes Wasser zu bilden. Zusätzlich zur Funktion als Auslaß für die abgeschiedenen Gase aus dem Wasser, dient das Lüftungsrohr 154 als Überlaufentleervorrichtung, um den Flüssigkeitsspiegel im ersten Speichertank unterhalb eines vorausbestimmten Niveaus zu halten. Zu diesem Zweck kann das Lüftungsrohr 154 z.B. 13 cm oberhalb des Bodens des 18 cm hohen ersten Speicherbehälters liegen.

In Verbindung mit der Funktion als Überlauf kann das Entlüftungsrohr 154 mit einem "Siphon Breaker" (nicht gezeigt) ausgerüstet sein, um den Rückfluß von überflüssigem Wasser in den ersten Speichertank 153 zu verhindern.

Im Betrieb ist die Flußsteuerung 151 so eingestellt, daß sie eine Volumswasserdurchflußrate von 10-20 % größer als die im Dialysatlösungs-Kreislauf des Hemodialysesystems benötigte vorsieht. Z.B. kann die Flußsteuerung 151 so eingestellt sein, daß sie den Wasserfluß in der Leitung 129 auf einem Wert von 550 ml/min reguliert, sodaß überschüssige Flüssigkeit mit einer Flußrate von bis zu 40 ml/min über den Überlauf des Entlüftungsrohres 154 ausfließt. Der geringfügige Überfluß von erwärmten Wasser für den ersten Speicherbehälter 153 ist vorgesehen, um sicherzustellen, daß die Wasserspeisung im ersten Speisebehälter sich im Betrieb nicht erschöpft. Ohne den Überschuß müßte die Flußrate von erhitztem Wasser in den ersten Speicherbehälter sich an den Fluß des Wassers in der Dialysatlösung, welche vom Bemessungssystem durch die Pumpen in den Dialysalösungs-Kreislauf gepumpt wird, anpassen, wobei diese Anpassung in der Praxis sehr schwierig kontinuierlich zu erzielen ist. Durch den vorgesehenen Überlauf zur Aufrechterhaltung eines Flüssigkeitsniveaus im ersten Speicherbehälter unterhalb eines vorausbestimmten Niveaus und der Einführung von 10-20 % Überschuß an Flüssigkeit in den ersten Speicherbehälter 153, ermöglicht die Überflußrate für eine entsprechende Fluktuation von 10-20 % in der Flußrate der Dialysatlösung, welche von der Pumpe in den Dialysatlösungs-Kreislauf ausgegeben wird. Auf diese Weise werden die Veränderungen in der Flußrate der Dialysatlösung von der Pumpe im Dialysatlösungs-Kreislauf durch einen korrespondierend geänderten Wasserzufluß für die Herstellung der Dialysatlösung ausgeglichen. Nichtsdestoweniger kann in bestimmten Anwendungen der vorliegenden Erfindung eine Änderung in der Flußrate im Dialysatlösungs-Kreislauf des Hemodialysesystems tolerierbar sein, sodaß der Überschuß-Zufluß in den ersten Speicherbehälter 153 und die Verwendung der Entlüftungsleitung 154 als Überlaufvorrichtung zur Aufrechterhaltung eines Flüssigkeitsspiegels im ersten Speicherbehälter unterhalb eines vorherbestimmten Niveaus nicht notwendig sind.

Das teilweise entlüftete Wasser im ersten Speichertank 153 wird durch die Leitung 155 abgeleitet. In dieser Leitung ist eine einstellbare Durchflußbegrenzung 156 angeordnet, um im durch die Leitung strömenden teilweise entlüfteten Wasser für eine weitere Gasabscheidung den Druck zu reduzieren und ein Wasser mit niedrigem Druck und mit darin verteiltem abgeschiedenem Gas zu bilden. Eine geeignete Flußbegrenzungsvorrichtung für diesen Zweck ist das Neu-trol Nadelventil 1/8 Zoll, EFL 10B, hergestellt von "Deltrol Corporation, Bellwood, Illinois". Die Flußbegrenzungsvorrichtung ist so eingestellt, daß sie einen bemerkbaren Druckabfall in der Flüssigkeit erzeugt, so z.B. von 600-750 mm Hg Druck in der von der Flußbegrenzungsvorrichtung ausgegebenen Flüssigkeit, bei einer Flußrate für Wasser durch die Leitung 155 von 600-750 ml/min. Der Zweck der einstellbaren Flußbegrenzungsvorrichtung 156 besteht darin, daß der Druck der in der Leitung 155 fließenden Flüssigkeit unterhalb des atmosphärischen Druckniveaus des teilweise entlüfteten Wassers, welches vom ersten Speicherbehälter 153 abgezogen wird, gesenkt wird, um durch die Drucksenkung in der Flüssigkeit ein weiteres Ablösen von lösbaren Gasen zu erzielen. Stromab der einstellbaren Flußbegrenzungsvorrichtung 156 ist eine Vakuumanzeigevorrichtung 157 angeordnet, welche in geeigneter Weise einen konventionellen Vakuummesser aufweisen kann. Derartige Anzeigevorrichtungen erlauben es, den Flüssigkeitsdruck des von der einstellbaren Flußbegrenzungsvorrichtung abfließenden Wassers genau zu bestimmen, sodaß jede notwendige Einstellung der Flußbegrenzung 156 schnell durchgeführt werden kann, um ein vorherbestimmtes Druckniveau im abfließenden Wasser aufrechtzuerhalten.

Das abschließende Ende der Leitung 155 ist mit dem Einlaß einer Vakuumpumpe 158 verbunden. Die verwendete Bezeichnung Vakuumpumpe bezieht sich auf eine positive Verlagerungspumpe, die geeignet ist, im Vakuumbetrieb zu arbeiten, d.h. Flüssigkeit mit einem Druck unterhalb des normalen Atmosphärendrucks aufzunehmen und diese auf einen höheren Druck zu pumpen. Die Pumpe 158 ist vorzugsweise eine Type, welche magnetisch gekuppelt ist und daher lecksicher und frei von Beeinträchtigung durch das zu pumpende
Wasser ist. Eine geeignete Pumpe ist die Zahnradpumpe
Modell 12-50-316, hergestellt von "Micropump Incorporated,
Concord, California". Wie erwähnt, arbeitet die Pumpe 158
im Vakuumbetrieb und pumpt 60-750 ml Wasser/min bei einem
Druck von z.B. 740 mm Hg Negativdruck in der in den Pumpeneinlaß einströmenden Flüssigkeit. Vom Einlaßdruck von
600-750 mm Hg wird die Flüssigkeit in der Pumpe 158 bis zu
einem Druck verdichtet, der etwa normalem Atmosphärendruck
entspricht. Mit dem Auslaß der Vakuumpumpe 158 ist eine
Leitung 159 verbunden, zur Ableitung von Wasser mit höherem
Druck und mit darin verteiltem abgeschiedenem Gas von der
Pumpe. Mit dem anderen Ende der Leitung 159 ist ein zweiter
Speicherbehälter 160 verbunden, zur Aufnahme des abgeleiteten Wassers mit höherem Druck. Der zweite Speicherbehälter
ist mit einer Entlüftungsvorrichtung versehen, um das
abgeschiedene Gas vom Wasser mit höherem Druck loslösen zu
lassen und endgültig entlüftetes Wasser zu bilden. Die
Entlüftungsvorrichtung kann in geeigneter Weise eine Entlüftungsleitung 167 vom Speicherbehälter mit einem von
Hand aus schließbaren Entlüftungsventil 168 sein.

Der zweite Speicherbehälter 160 kann dieselbe Größe und
Abmessungen wie der erste Speicherbehälter 153 aufweisen,
sodaß z.B. der erste Speicherbehälter ein zylindrisches
Gefäß mit 5 cm Durchmesser und 18 cm Höhe aufweisen kann.
Obwohl der erste Speicherbehälter 153 und der zweite Speicherbehälter als voneinander getrennte Behälter vorgesehen
sein können, können die beiden Behälter auch konzentrisch
zueinander ausgebildet sein, mit einem kleineren zweiten
Behälter innerhalb eines größeren ersten Speicherbehälters.
Bei dieser konzentrischen Anordnung ist das Volumen des
inneren zweiten Speicherbehälters gleich dem Ringvolumen
zwischen den benachbarten Wänden des ersten und zweiten
Speicherbehälters. Eine derartige konzentrische Anordnung
kann wünschenswert sein, wenn eine geringe Größe und Kompaktheit des gesamten Bemessungssystems 126 erzielt werden soll. Vom zweiten Speicherbehälter 160 wird das endgültig entlüftete Wasser in die Leitung 162 zu der stromab gelegenen Portioniereinrichtung geleitet.

Um die Entfernung von lösbaren Gasen durch die oben beschriebene Entlüftungsvorrichtung zu erhöhen, ist eine Leitung 161 vorgesehen, die den ersten Speicherbehälter 153 mit dem zweiten Speicherbehälter 160 verbindet, um einen Teil des endgültigen entlüfteten Wassers vom zweiten Speicherbehälter zu dem ersten Speicherbehälter zurückzuleiten. Eine derartige Rückführleitung stellt kein wesentliches Merkmal des Entlüftungssystems dar, wird aber in der Praxis gerne zur Erhöhung der Entfernung von lösbaren Gasen im Entlüftungssystem verwendet. Mit der vorhin beschriebenen beispielsweisen Wasserflußrate von 600-750 ml/min in der Leitung 155 und von hier in die Leitung 159, welche in den zweiten Speisebehälter führt, werden ca. 20-50 % des den zweiten Speicherbehälter 160 über die Leitung 159 betretenden Wassers in der Leitung 161 zu dem ersten Speicherbehälter 153 zurückgeführt, sodaß die Flußrate von endgültig entlüftetem Wasser, welches vom zweiten Speicherbehälter 160 in die Leitung 162 ausgegeben wird, geeigneterweise eine Größe von 110 ml/min liegt weil, wenn die Rückführleitung 161 verwendet wird, die Flußrate des Wassers in der Leitung 159 größer als die Flußrate des endgültig entlüfteten Wassers abgegeben in der Leitung 162, eingestellt ist, ist die Flußrichtung durch die Leitung 161 immer vom zweiten Speicherbehälter zum ersten Speicherbehälter 153 gerichtet. Wie erwähnt, werden 20-50 % des dem zweiten Speicherbehälter 160 in der Leitung 159 zugeführten Wassers in der Leitung 161 zum ersten Speicherbehälter 153 zurückgeführt, von dem die zurückgeführte Flüssigkeit einer weiteren Entlüftung beim Fließen durch die Leitung 155, welche einstellbare Flußbegrenzungsmittel 156 aufweist, durch die Pumpe 158 und die Leitung 159 zur Rückführung zu dem entlüfteten zweiten Speicherbehälter 160 unterzogen wird.

Während beide, der erste Speicherbehälter 153 und der zweite Speicherbehälter 160 zur Atmosphäre entlüftet sind, ist der zweite Speicherbehälter 160 mit einem absperrbaren Ventil ausgerüstet so z.B. mit dem von Hand aus verschließbaren Entlüftungsventil 168, das in der Entlüftungsleitung 167 angeordnet ist. Das Vorsehen von verschließbaren Ventilen ist insoferne wünschenswert, als sie ein Trockenlegen des ersten und zweiten Speicherbehälters erlauben, wenn das Bemessungssystem nicht mehr verwendet wird. Das Trockenlegen wird durchgeführt durch das Entleeren des Inhalts des zweiten Speicherbehälters 160 und dann des ersten Speicherbehälters 153 nacheinander. Um die Trockenlegung durchzuführen, wird das von Hand aus schließbare Entlüftungsventil 168 in der Entlüftungsleitung 165 des zweiten Speicherbehälters 160 geschlossen und der Fluß des einfließenden Wassers zu dem Bemessungssystem in der Leitung 129 wird zusammen mit der Vakuumpumpe 158 unterbrochen, während die Dialysatlösungspumpe im Dialysatlösungs-Kreislauf des Hemodialysemoduls im Betrieb gehalten wird. Durch die Funktion der Pumpe im stromab gelegenen Abschnitt des Dialysatlösungs-Kreislaufes wird der Flüssigkeitsinhalt des zweiten Speicherbehälters 160 durch die Abflußleitung 162 abgezogen. Wenn der zweite Speicherbehälter entleert ist, wird das im ersten Speicherbehälter verbliebene Wasser in den zweiten Speicherbehälter über die Verbindungsleitung 161 gezogen, u.zw. wegen der Druckdifferenz zwischen den beiden Speicherbehältern.

Vom zweiten Speicherbehälter 160 überträgt die Leitung 162 das endgültig entlüftete Wasser zur Proportioniereinrichtung, die ein Mischventil 163 aufweist. Im Mischventil 163 wird das endgültig entlüftete Wasser mit dem Dialysatkonzentrat in einem vorbestimmten Verhältnis gemischt, um die Dialysatlösung für die Hemodialyse zu bilden. Das Dialysatkonzentrat wird dem Mischventil von einem Dialysatkonzentratbehälter 127 zugeführt, von dem das Dialysatkonzentrat in einer Leitung 128 mittels einer Pumpe 164 abgezogen und dem Mischventil 163 zugeführt wird. Wenn das Wasser und das Dialysatkonzentrat miteinander im Mischventil 163 gemischt sind, wird die sich ergebende Dialysatlösung vom Mischventil in die Leitung 165 abgegeben und durch eine Leitfähigkeitsanzeigevorrichtung 166 geleitet. Die Leitfähigkeitsanzeigevorrichtung 166 kann mit einer sichtbaren Anzeige verbunden sein, die die gemessene elektrolytische Leitfähigkeit der Dialysatlösung anzeigt, sodaß der Patient oder Benützer des Systems rasch eine Veränderung im Wert der elektrolytischen Leitfähigkeit der Dialysatlösung von einem gewünschten Wert weg feststellen, und die Geschwindigkeit der Dialysatkonzentratpumpe 164 entsprechend einstellen kann, um das relative Verhältnis von Wasser und Dialysatkonzentrat, welche miteinander gemischt werden, zu ändern, sodaß der Wert der elektrolytischen Leitfähigkeit der vom Mischventil ausgegebenen Dialysatlösung auf den gewünschten Wert eingestellt wird. Alternativ dazu kann die Leitfähigkeitsanzeigevorrichtung 166 mit der Dialysatkonzentratpumpe 164 gekuppelt sein, um z.B. mittels eines Rückkopplungskreises, der bewirkt, daß die Geschwindigkeit der Pumpe 164 eingestellt wird, um das relative Verhältnis von endgültig entlüftetem Wasser und dem zu mischenden Dialysatkonzentrat in Abhängigkeit von der gefühlten Abweichung von einem eingestellten Wert für die elektrolytische Leitfähigkeit zu ändern, um die elektrolytische Leitfähigkeit auf einem vorbestimmten Wert zu halten. Das Mischungsverhältnis des endgültig entlüfteten Wassers und des Dialysatkonzentrates im Mischventil 163 kann bei einem Volumenverhältnis von ungefähr 34:1 liegen, korrespondierend z.B. mit einer Flußrate von endgültig entlüftetem Wasser in der Leitung 162 von 510 ml/min und einer Flußrate des Dialysatkonzentrats in der Leitung 128 zum Mischventil von 15 ml/min zur Bildung einer Dialysatlösung ausgegeben in der Leitung 165 vom Mischventil 163 bei einer Flußrate von 525 ml/min und einer Temperatur von 29,4 bis 35°C und ungefähr Atmosphärendruck. Von der Leitfähigkeitsanzeigevorrichtung 166 wird die Dialysatlösung vom Bemessungssystem in die Leitung 130 ausgegeben und durch den Hemodialyse-Lösungskreislauf im Hemodialysemodul in der oben beschriebenen Weise geleitet. Ein Vorteil des oben beschriebenen Bemessungssystems besteht darin, daß es eine Dialysatlösung für das Hemolysesystem zu im wesentlichen immer gleichbleibenden Bedingungen, d.h. Druck, Temperatur und Leitfähigkeit vorsieht wie eine Dialysatlösung, die dem Hemodialysesystem von einem chargenweisen Speisecontainer zugeführt wird. Diese Identität der Dialysatlösung von entsprechenden Quellen erlaubt es, das Hemodialysemodul rasch und einfach von einer Quelle auf die andere - vom chargenweisen Speisecontainer zum Bemessungssystem und umgekehrt - umzustellen, ohne die Vorrichtung oder die Betriebsbedingungen im Hemodialysemodul zu ändern.

Fig. 4 ist eine Draufsicht auf eine peristaltische Dialysatlösungspumpe, verwendet in einem Hemodialysesystem gemäß Fig. 2, welche die Dimensionscharakteristika zeigt. Wie gezeigt, ist die Pumpe 116 in einem rechtwinkligen schalenförmigen Behälter 200 angeordnet. Die Pumpe 116 umfaßt einen Pumpenkopf der eine Pumpenkopfbasis 201 einschließt, die Pumpenkopfbasis 201 weist einen erhabenen zylindrischen Spindelabschnitt 203 auf, der über eine drehbare Welle, welche mit dem Antrieb für die Pume verbunden ist, aufgesetzt werden kann. Auf diese Weise ist die Pumpenkopfbasis 201 zur Drehung um eine fixe Achse angeordnet. An der Pumpenkopfbasis 201 sind mittels geeigneter Bolzen oder Schrauben für die Basis mit fixer Achse vier über den Umfang verteilte Rollen 202 a-d befestigt.

Der Dialysatlösungs-Kreislaufabschnitt, verbunden mit der Dialysatlösungspumpe, weist schlauchförmige Abschnitte 114 und 117 auf, welche durch eine Endwand des Behälters 200 führen und in Fittings 204 und 205 münden, welch letztere in geeigneter Weise an der Behälterendwand befestigt sind. Die Fittings 204 und 205 weisen mit Flanschen versehene weibliche Verbinder 206 und 207 auf. Mit den weiblichen Verbindern stehen männliche Verbinder 208, 209 in Eingriff. Die männlichen Verbinder werden durch nach innen gerichtete Fingerglieder 210 und 211, welche sich um die Flanschabschnitte der entsprechenden weiblichen Verbinder 206 und 207 schließen, in ihrer Stellung gehalten, sodaß die männlichen Verbinder sehr rasch von den weiblichen Verbindern abgezogen werden können, indem man auf die entsprechenden Finger der männlichen Verbinder drückt, um die sich nach innen erstreckenden Fingerabschnitte von den Flanschabschnitten der entsprechenden weiblichen Verbinder außer Eingriff zu bringen. Die männlichen Verbinder sind mit Widerhakenvorsprüngen 212 und 213 versehen, mit denen die entsprechenden Enden eines flexiblen elastischen Pumpleitungsabschnittes 214 durch den die Dialysatlösung gepumpt wird, verbunden sind. Die oben beschriebenen Verbinder und angeschlossenen Fittings dienen zur Verankerung der Endabschnitte des flexiblen elastischen Pumpleitungsabschnittes 214, sodaß die Leitung um den Pumpenkopf gespannt ist, und gleichzeitig im Eingriff steht und zusammengedrückt wird, von zumindest zwei der am Umfang verteilten Rollen, wobei die Rollen zur Längsbewegung der Druckpumpe entlang der Leitung während der Drehung des Pumpenkopfes zum Vorschieben der Dialysatlösung durch die Leitung befestigt sind.

Die Dialysatlösungspumpe, gezeigt in Fig. 4, stellt eine Verbesserung dar, welche unerwarteterweise entdeckt wurde, um einen im wesentlichen konstanten Fluß im Dialysatlösungskreislauf des Hemodialysesystems trotz der Änderungen im Negativdruck die auf die Dialysatlösung im Kreislauf für die Ultrafiltrations-Anforderungen des zu behandelnden Patienten aufgebracht werden, zu ermöglichen. Daraus ergibt sich, daß die Effizienz der Dialyse deutlich erhöht wird, wobei die Dialysatlösungspumpe einen gleichförmigen Fluß im Dialysatlösungs-Kreislauf auch bei hohem negativen Druckniveau aufrechterhält, sodaß die Dialysebehandlung innerhalb einer relativ kurzen Zeit auch bei hohen negativen Drücken ausgeführt werden kann.

Bei der Verbesserung, wobei die peristaltische Dialysatlösungspumpe vier auf der Pumpenbasis befestigte Rollen umfaßt, besitzt jede Rolle einen Maximaldurchmesser D gezeigt in Fig. 4, zwischen 6,3 und 19 mm und sind bezüglich des Umfangs voneinander um einen Winkel von 90° versetzt, wobei der radiale Abstand P zwischen der Rollenachse und der festen Achse der Pumpenkopfbasis 201 zwischen 12,7 und 38 mm liegt. Die Verbesserung erfordert auch einen flexiblen elastischen Pumpschlauchabschnitt mit einer Länge, gemessen entlang des Schlauches zwischen dessen verankerten Abschnitten, von 17,1 bis 28 cm und einer Wandstärke von 1,78 bis 3,18 mm und einem inneren Durchmesser von 4,57 bis 8,89 mm, wobei Antriebsmittel mit dem Pumpenkopf verbunden sind, zu dessen Drehung bei einer Geschwindigkeit von 150-400 1/min.

Obwohl peristaltische Pumpen zum Pumpen von Dialysatlösung im Hemodialysesystem-Dialysatlösungs-Kreisläufen bekannt sind, waren diese bekannten Pumpen nicht in der Lage, einen im wesentlichen gleichförmigen Fluß von der Pumpe unter den Bedingungen des sich ändernden negativen Drucks der Dialysatlösung zu erzielen. Die erfindungsgemäße Pumpe stellt eine wesentliche Verbesserung gegenüber der peristaltischen Pumpe, geoffenbart in US-S.No. 720 672, angemeldet 7.9.1976, unter dem Namen "J.T.Hutchisson" dar, welche nachfolgend genauer beschrieben wird.

Die peristaltische Pumpe, geoffenbart in der vorhin genannten US-S.No. 720 672, ist geeignet für das Pumpen einer Dialysatlösung im Dialysatlösungs-Kreislauf eines Hemodialysesystems und umfaßt drei Rollen, die auf einer Basis montiert sind, wobei jede Rolle einen Durchmesser zwischen 6,35 und 19 mm aufweist und die gegeneinander am Umfang in einem Winkel von 120° versetzt sind, wobei der radiale Abstand zwischen der Rollenachse und der fixen Achse des Pumpenkopfes zwischen 12,7 und 31,7 cm liegt. Diese bekannte Pumpe verwendet einen flexiblen elastischen Schlauchpumpenabschnitt, mit einer Länge gemessen längs des Schlauches zwischen den verankerten Enden von 15,24 bis 17,15 cm, einer Wandstärke von 0,76 bis 2,5 mm und einem inneren

0004600

Durchmesser von 4,57 bis 6,35 mm, wobei der Pumpenkopf mit einem Antrieb verbunden ist, für dessen Drehung eine Geschwindigkeit im Bereich von 100-600 1/min vorgesehen ist.

Obwohl die Pumpe gemäß der vorliegenden Erfindung strukturell ähnlich der bekannten Pumpe der US-S.No. 720 672 in vielen Fällen ist, verkörpert die Pumpe gemäß vorliegender Erfindung mehrere wesentliche strukturelle Unterschiede gegenüber der bekannten Pumpe, welche eine wesentliche Verbesserung der Gleichförmigkeit der Dialysatlösungs-Flußrate bei Änderung des negativen Druckes in der Dialysatlösung erzielt. Diese wesentlichen strukturellen Unterschiede der erfindungsgemäßen peristaltischen Pumpe gegenüber der bekannten Pumpe US.S.No. 720 672 umfassen die Verwendung einer größeren Anzahl von Rollen, wobei die Rollen eine im allgemeinen konvexe Profilfläche im Gegensatz zur ebenen Profilfläche der bekannten Pumpe vorsehen und die Verwendung einer im allgemeinen größeren Länge des flexiblen elastischen Schlauchpumpenabschnittes.

Um den erzielten Fortschritt der erfindungsgemäßen peristaltischen Pumpe vollständig zu erfassen, ist es zuerst notwendig, die Effekte der Veränderungen des negativen Drucks der Dialysatlösung auf die Ausflußrate der Dialysatlösung von der bekannten peristaltischen Pumpe angeordnet in einem Dialysatlösungs-Kreislauf zu erörtern. Im Dialysatlösungs-Kreislauf wird ein Pumpenabschnitt aus einem flexiblen elastischen Schlauch im Dialysatlösungs-Kreislauf um einen peristaltischen Pumpenkopf unter Spannung angeordnet, welcher simultan im Eingriff steht und zusammengedrückt wird, durch zumindest zwei am Umfang voneinander im Abstand angeordneten Rollen. Die Rollen sind befestigt, zur Längsbewegung der Punkte des Zusammendrückens längs des Schlauches während der Drehung des Pumpenkopfes, um die Dialysatlösung durch den Schlauch zu befördern. An den Kontaktpunkten des Pumpschlauchabschnittes mit den Pumpenkopfrollen drücken die Rollen den Schlauch zusammen und ändern seinen Querschnitt vom normalen Kreisquerschnitt des ungepreßten Schlauches. Das Ausmaß der Reduktion der Querschnittsfläche des Schlauches am Kontaktpunkt mit der Rolle wird im allgemeinen charakterisiert als Grad der Occlusion des elastischen flexiblen Schlauchpumpenabschnittes.

Im allgemeinen ist die Effizienz einer peristaltischen Pumpe eine Funktion des Grades der Occlusion für den Schlauch und des Flüssigkeitsvolumens, das im Schlauchabschnitt zwischen benachbarten Rollenkompressionspunkten eingeschlossen ist. Das Flüssigkeitsvolumen im Schlauchabschnitt zwischen benachbarten Rollendruckpunkten ist eine Funktion der lokalen Querschnittsfläche des Schlauches entlang seiner Länge zwischen den benachbarten Druckpunkten, welche abhängig ist vom Grad der Occlusion und der Länge des Schlauches zwischen benachbarten Druckpunkten, welche abhängig ist vom Grad der Occlusion und der Länge des Schlauches zwischen benachbarten Druckpunkten. Die örtliche Querschnittsfläche des Schlauchabschnittes zwischen benachbarten Rollen hängt ab vom Grad der Occlusion an den Rollendruckpunkten, insofern, als der Rollendruck am Kontaktpunkt den Schlauchabschnitt abflacht und dieser Abflacheffekt am Schlauch etwas von der Rolle entfernt ausgeübt wird. Die Pumpeffizienz der peristaltischen Pumpe wird auch direkt beeinflußt durch den Grad der Occlusion des Schlauchabschnittes infolge der Tatsache, daß jede weniger als vollständige Occlusion des Schlauches einen Rückfluß von Flüssigkeit vom Schlauchabschnittteil stromab der Rolle zum Schlauchabschnittteil stromauf zur Rolle zuläßt, durch den nicht abgeschlossenen Querschnitt des Schlauches am Druckpunkt. Deshalb sah die bekannte Pumpe gemäß US-S.No. 720 672, wenn sie für das Pumpen einer Dialysatlösung verwendet wurde, einen relativ kurzen Pumpenschlauchabschnitt und eine hohe Spannung im Schlauchabschnitt, wenn sich dieser um den Pumpenkopf erstreckt, vor, um eine vollständige Occlusion, d.h. einen totalen Verschluß des Schlauches an den Kontaktpunkten mit den Rollen zu erzielen. Diese vollständige Occlusion an den Druckpunkten des Schlauches mit den Rollen wurde angewendet, um die Pumpeneffizienz zu maximieren, wobei der Rückfluß vom Tubenabschnitteil stromab der Rolle zum Tubenabschnitteil stromauf der Rolle verhindert wird und eine im wesentlichen vollständige positive Verlagerung der Flüssigkeit, die im Schlauchabschnitt zwischen benachbarten Rollen enthalten ist, erzielt wird.

Trotz der vollständigen Occlusion des Schlauchabschnittes an den Punkten des Kontaktes mit den Rollen der bekannten Punkte gemäß US-S.No. 720 672, welche mutmaßlich eine hohe Pumpeneffizienz vorsehen würde, hat es sich herausgestellt, daß die Ausflußrate der Dialysatlösung mit dieser bekannten Pumpe bei ansteigendem negativen Druck drastisch abfällt. Eine derartige Flußrate mit negativer Druckcharakteristik stellt einen schweren Betriebsnachteil für die bekannte Pumpe dar, wenn diese zum Pumpen einer Dialysatlösung mit einem Hemodialysesystem verwendet wird, weil es im allgemeinen wünschenswert ist, die Höhe des negativen Drucks auf die Dialysatlösung, wenn diese durch den Dialysator zur Dialysebehandlung von Patient zu Patient und für einen bestimmten Patienten von Behandlung zu Behandlung zu verändern. Der Grund für die Änderung des negativen Drucks der Dialysatlösung für die Dialyse von Patient zu Patient oder von Behandlung zu Behandlung ist die Einstellung der Druckgradientenantriebskraft für die Massenübertragung von Wasser vom Blut durch die Massenübertragungsfläche des Dialysators zur Dialysatlösung, welche in indirekter Massenübertragungs Dialysebeziehung mit dem Blut geleitet wird, auf ein geeignetes Niveau, um die im wesentlichen vollständige Entfernung von Wasser aus dem Blut während der Dialysebehandlung sicherzustellen. In dieser Beziehung reagiert die oben beschriebene bekannte Pumpe auf ein Ansteigen des negativen Drucks mit einer drastisch reduzierten Ausflußrate der Dialysatlösung von der Pumpe. Die daraus resultierende Reduktion der Flußrate der Dialysatlösung im Dialysatlösungs-Kreislauf des Hemodialysesystems läuft der insgesamten Effizienz der Dialysebehandlung entgegen und erfordert ein deutliches Ansteigen der Behandlungszeit, um die notwendige Entfernung von Verunreinigungen aus dem zu behandelnden Blut zu bewirken.

Der Grund für die oben beschriebene drastische Reduktion der Ausflußrate der Dialysatlösung mit dem Ansteigen des negativen Drucks der Dialysatlösung bei der bekannten Pumpe steht in Verbindung mit der Anwendung der vollständigen Occlusion des Pumpenschlauchabschnittes an der Kontaktstelle mit der Rolle, welche komischerweise zur Erhöhung der Pumpeneffizienz angewendet wird. Wie oben bereits ausgeführt, resultiert aus der Dehnung des Pumpenschlauchabschnittes um die Rollen des Pumpenkopfes einer peristaltischen Pumpe im Abflachen der Schlauchquerschnitte, die sich entlang der Länge des Schlauchabschnittes vom Druckpunkt des Schlauches gegen die Rolle weg erstrecken. Das längliche Ausmaß einer solchen Abflachung entlang des Schlauches ist eine Funktion des Grades der Occlusion, wobei der Grad der Abflachung und das längliche Ausmaß des Abflachungseffektes entlang dem Schlauchabschnitt am größten ist, wenn der Schlauchabschnitt an der Kontaktstelle mit der Rolle vollständig verschlossen ist. In einem derartigen Pumpensystem mit vollständiger Occlusion, wenn ein ansteigender negativer Druck auf die in dem Pumpschlauchabschnitt einströmende Flüssigkeit aufgebracht wird, wirkt der atmosphärische Druck an der äußeren Oberfläche des Schlauchabschnittes und wird entsprechend größer als der an der inneren Oberfläche des Schlauchabschnittes wirksame Druck, wenn die Flüssigkeit durchfließt. Diese Druckdifferenz führt zu einem Zusammenfallen des Schlauches von jener Gestalt, welche im anderen Fall annehmen würde, wenn keine derartig Druckdifferenz bestünde. Der Schlauchabschnitt, in dem eine vollständige Occlusion aufweisenden Pumpsystem, ist insbesondere für weiteres Zusammenfallen anfällig, u.zw. infolge der Tatsache, daß ein wesentlicher Teil der Länge des Schlauchpumpenabschnittes bereits zu einem größeren Teil abgeflacht ist, als Folge der vollständigen Occlusion des Schlauches an den Kontaktpunkten mit den Rollen, wie oben erwähnt. Mit anderen Worten, die Deformation des Schlauch-

0004600

schnittes infolge des negativen Druckunterschiedes ist in einem bereits abgeflachten Schlauchabschnitt größer als in einem undeformierten Schlauchabschnitt mit normalerweise Kreisquerschnitt. Aus diesem Grunde fallen die Schlauchabschnittsteile zwischen benachbarten Rollen in dem Pumpsystem mit vollständiger Occlusion sehr häufig zusammen, wenn der negative Druck in der Dialysatlösung ansteigt. Das Zusammenfallen der Schlauchabschnittsteile zwischen benachbarten Rollen verringert das Flüssigkeitsvolumen das in einem derartigen Schlauchsegment enthalten ist, wesentlich, sodaß die Menge der durch den Schlauch von der Pumpe weitertransportierten Dialysatlösung entsprechend verringert wird. Aus diesem Grund setzt das Dialysatlösungspumpsystem mit vollständiger Occlusion die Flußrate der Dialysatlösung von der Pumpe einer drastischen Reduktion aus, wenn das Niveau des negativen Drucks der Dialysatlösung ansteigt.

In Anbetracht der oben beschriebenen Unzulänglichkeit des bekannten Pumpsystems könnte man annehmen, daß die Verwendung von relativ dickwandigen Schläuchen das Problem der geringen Flußraten bei hohen negativen Druckniveaus mildern würde, weil ein relativ dicker Schlauchabschnitt weniger anfällig auf die Verformungen durch die aus dem negativen Druck der Dialysatlösung resultierenden Druckunterschiede wäre. Trotz dieses offensichtlichen Vorteils sind relativ dickwandige Schläuche für ihre Verwendung in einem Pumpsystem nicht geeignet, insofern, als es entsprechend schwieriger ist, diese für ein effektives Pumpen in geeigneter Weise zusammenzudrücken. In diesem Zusammenhang sei darauf hingewiesen, daß zumindest eine geringfügige Occlusion des Schlauchabschnittes an den Druckpumpen mit den Rollen vorhanden sein muß, ansonsten das Pumpsystem zum Weitertransport der Dialysatlösung durch den Schlauch nicht funktionieren würde. Darüberhinaus bestand die Lehre des Standes der Technik darin, den Schlauchabschnitt an dem Druckpunkt mit den Rollen vollständig zusammenzudrücken, was entsprechend schwieriger ist als nur ein teilweises Zusammendrücken. Aus diesem Grund ist die Verwendung einer Schlauchwandstärke wesentlich größer, als jene bei der bekannten Pumpe, d.h. wesentlich größer als 2,54 mm, keine geeignete Lösung für das vorhin genannte Problem.

Relativ zur peristaltischen Pumpe der bekannten Art sieht die Dialysatlösungspumpe der Erfindung eine unerwartete Verbesserung der in Form eines im wesentlichen konstanten Ausflusses von Dialysatlösung trotz des Ansteigens des negativen Drucks der zu pumpenden Dialysatlösung vor. Diese überraschende Verbesserung ist die Folge mehrerer bestimmter miteinander in Verbindung stehender Pumpcharakteristika wie nachfolgend erläutert.

Erstens: Relativ zur oben beschriebenen bekannten Pumpe weist die Pumpe der Erfindung vier auf einer Pumpenbasis montierte Rollen auf im Vergleich zu den drei Rollen der bekannten Pumpe. Das Vorsehen einer zusätzlichen Rolle verringert die Länge des Pumpabschnitteiles zwischen einander benachbarten Rollen gegenüber dem bekannten System, sodaß die Pumpabschnitteile zwischen benachbarten Rollen entsprechend steifer sind und einen größeren Widerstand gegen Deformationen haben, wobei die negativen Druckkräfte dieses Schlauchsegmentes in einem viel geringeren Ausmaß zusammendrücken, als die Schlauchsegmente bei dem bekannten Pumpsystem.

Zweitens: Die bei der Pumpe gemäß der Erfindung verwendeten Rollen haben ein im allgemeinen konvexes Profil und sind geeignet, den Schlauch an den Druckstellen der Rollen nur teilweise zusammenzupressen. Bei einer derartigen Rollengestalt der Pumpe gemäß der Erfindung im Gegensatz zur bekannten Pumpe, welche Rollen mit ebenem Profil vorsieht, wird eine vollständige Occlusion des Schlauchabschnittes an der Druckstelle mit der Rolle vermieden, wie sie sonst zu einer übermäßigen Abflachung des Schlauchabschnittes zwischen benachbarten Rollen führen würde, und diese Schlauchabschnitte überproportional anfälliger für eine Deformation infolge der negativen Druckkräfte machen würde. In dieser Beziehung beschränkt das im allgemeinen konvexe Profil der Rollen die Deformation des Schlauchquerschnittes auf einen schmalen Bereich knapp neben dem Druckpunkt mit der Rolle im Gegensatz zum bekannten System, indem die ebenen Rollen und die vollständige Occlusion in den Druckpunkten mit den Rollen in einer deutlichen Abflachung der Schlauchabschnitte über einen merkbaren Abstand vom Kontaktpunkt mit den Rollen wegführen.

Drittens: Die Pumpe gemäß der Erfindung verwendet einen flexiblen elastischen Schlauchpumpenabschnitt mit einer Länge, welche im allgemeinen größer als die im bekannten Pumpsystem verwendete Länge ist. Dieser Längenunterschied bedeutet einen deutlichen Unterschied in der Spannung der um die Rollen des Pumpenkopfes ausgedehnten Schlauchabschnitte. Die Längen der Schlauchpumpenabschnitte sind im allgemeinen mit einer speziellen Dehnungscharakteristik verbunden, für den um die Pumpenkopfrollen gestreckten Schlauchabschnitt, wobei kürzere Längen des Schlauchpumpenabschnittes im allgemeinen mit einem höheren Spannungsniveau verbunden sind und größere Pumpenschlauchabschnitte im allgemeinen mit geringeren Spannungsniveaus. Das bekannte Pumpensystem verwendet relativ kurze Pumpschlauchabschnitte, welche mit relativ hohen Spannungsniveaus verbunden sind, welche verwendet werden, um eine vollständige Occlusion des Schlauchabschnittes am Kontaktpunkt mit den Rollen zu erleichtern. Im Gegensatz dazu, verwendet die Pumpe gemäß der Erfindung eine relativ größere Länge für den Schlauchpumpenabschnitt, was mit relativ geringeren Spannungsniveaus verbunden ist, um nur eine teilweise Occlusion oder Schließung des Schlauches an den Druckpunkten mit den Rollen vorzusehen.

Mehr im allgemeinen ist der spezielle Längenbereich des elastischen flexiblen Schlauchpumpenabschnittes gemäß der Erfindung verbunden mit der Aufgabe, bei einer vergleichsweise geringeren Rotationsgeschwindigkeit des Pumpenkopfes zu arbeiten, als die oben beschriebene bekannte Pumpe, um eine relativ größere Lebensdauer für den Schlauchpumpenabschnitt vorzusehen, indem das Ausmaß des Kontaktes zwischen dem Schlauch und den Pumpenkopfrollen reduziert wird und ein ruhigerer Betrieb erzielt wird. Zur Lösung der Aufgabe bei geringerer Rotationsgeschwindigkeit zu arbeiten, obwohl die Größen- und Gewichtsanforderungen in Verbindung mit der Transportierbarkeit des Hemodialysesystems diesem anzupassen sind, wurde der Abstand der Rollen (Entfernung zwischen der Achse der Rollen und der fixen Achse der Pumpenkopfbasis) auf den größten durchführbaren Wert vergrößert, übereinstimmend mit dem Kraftverbrauch des Antriebes und den Drehmomentüberlegungen, wobei auf diese Weise die Anforderungen an die Schlauchlänge gemäß der vorliegenden Erfindung erstellt wurden. Gemäß den oben genannten strukturellen Merkmalen weist der Pumpenschlauchabschnitt der Pumpe gemäß der vorliegenden Erfindung an den Stellen des Zusammendrückens durch die Rollen nur eine teilweise Occlusion auf.

Eine derartige teilweise Occlusion des Schlauchabschnittes läßt es zu, daß die Ausflußrate der Dialysatlösung von der Pumpe im wesentlichen konstant gehalten wird, obwohl der negative Druck der zu pumpenden Dialysatlösung ansteigt. Im Betrieb, wenn ein negativer Druck in der Dialysatlösung ansteigt, wird ein steigender Druck an die äußere Wandfläche des Schlauchpumpenabschnittes ausgeübt, welcher die Teile des Pumpenabschnittes zwischen benachbarten Rollen in einem bestimmten Maß deformiert und das Volumen dieser Schlauchteile verringert, wie dies auch in dem oben beschriebenen bekannten Pumpensystem auftritt. Bei der Pumpe gemäß der vorliegenden Erfindung tritt eine Deformation des Schlauchquerschnittes als Resultat der ansteigenden negativen Druckdifferenz auch im Schlauch an den Stellen der Zusammendrückung durch die Rollen auf, wodurch ein Ansteigen der Occlusion des Schlauches an diesen Druckpumpem hervorgerufen wird. Der ansteigende Grad der Occlusion an den Druckpunkten reduziert entsprechend den Rückfluß von dem Teil des Schlauchabschnittes stromab der Rolle, zu dem Teil des Schlauchabschnittes stromauf der Rolle, sodaß die Pumpeneffizienz ansteigt. Der vorteilhafte Effekt der ansteigenden Occlusion als Steigerung der Pumpeneffizienz bei höheren negativen Druckniveaus hat sich herausgestellt als Ausgleich für die Volumsreduktion des Schlauchabschnittes zwischen benachbarten Rollen als Ergebnis des ansteigenden negativen Druckes, sodaß diese einander widersprechenden Effekte im wesentlichen ausgeglichen werden und so eine im wesentlichen gleichbleibende Ausflußrate der Dialysatlösung aus der Pumpe trotz des Anstieges des negativen Drucks in der zu pumpenden Dialysatlösung vorsehen.

Gemäß der vorliegenden Erfindung muß der peristaltische Pumpabschnitt des Schlauches sowohl flexibel, als auch elastisch sein, sodaß der Schlauch auch unter kontinuierlich sich ändernden Dehnungs- und Druckbeanspruchungen im Gebrauch nicht ermüdet oder bricht und daß der Schlauch sehr rasch seine unverformte Gestalt und Dimension annimt, nachdem die direkte Beanspruchung des Drucks durch die Rollen vom Schlauch entfernt wurde, wenn sich die Rolle entlang der Schlauchlänge bewegt. Es hat sich herausgestellt, daß Pumpenabschnitte aus Siliconelastomeren, insbesondere aus Siliconelastomeren mit einer Durometeranzeige von 40-60, die vorstehend genannten Bedingungen erfüllen und für die Verwendung in der vorliegenden Erfindung besonders geeignet sind. Gemäß der vorliegenden Erfindung weisen die am Pumpenkopf montierten Rollen eine im wesentlichen konvexe Profilfläche auf, mit einem Maximaldurchmesser zwischen 6,35 und 19 mm. Bei Durchmesserwerten unter 6,35 mm wird es zunehmend schwierig, eine nur teilweise Occlusion des Schlauchpumpenabschnittes, wie er erfindungsgemäß erforderlich ist, zu erzielen. Steigt der Durchmesser der Rollen über 19 mm, so wird ein sehr großer Schlauchbereich einer teilweisen Occlusion unterworfen, wenn die Rolle in den Schlauchpumpenabschnitt eingreift, wodurch das augenblickliche Pumpvolumen der Pumpe in unerwünschter Weise gesenkt wird und damit auch die Pumpeneffizienz der Pumpe.

Die vorliegende Erfindung erfordert einen radialen Abstand gemessen zwischen einer bestimmten Rollenachse und der fixen Achse der Pumpenkopfbasis zwischen 12,7 und 38 mm. Der Grund für derartige Grenzen ist zu den oben angeführten Gründen für die Begrenzung der Rollendurchmesser komplementär. Wenn der radiale Abstand größer als 12,7 mm ist, so steigt die Rotationsgeschwindigkeit, die zur Aufrechterhaltung einer geeigneten Ausflußrate an Dialysatlösung notwendig ist. Dazu kommt noch, daß bei radialen Abständen kleiner als 12,7 mm, ein steigender Bereich des Schlauches einer teilweisen Occlusion unterworfen wird, wenn die Rollen in den Schlauchpumpenabschnitt eingreift. Dadurch wird das augenblickliche Pumpvolumen der Pumpe und damit die Pumpeneffizienz in unerwünschter Weise verringert. Andererseits, wenn der radiale Abstand größer als 38 mm ist, werden die nicht unterstützten Längen des Schlauches zwischen benachbarten Rollen relativ zur Größe der Rollen, die den Schlauch berühren zu lange, sodaß die nicht unterstützten Längen des Schlauches überproportional anfällig sind bezüglich der Deformationen aufgrund Druckdifferenzkräften in Verbindung mit höheren negativen Druckniveaus in der Dialysatlösung.

Der flexible elastische Schlauchpumpenabschnitt gemäß der vorliegenden Erfindung muß eine Länge aufweisen, die gemessen entlang des Schlauches zwischen dessen verankerten Enden von 17,15 bis 28 cm beträgt und eine Wandstärke von 1,7 bis 3,2 mm und einen Innendurchmesser von 4,5 bis 8,9 mm aufweisen. Wenn die Länge des Schlauchpumpenabschnittes, gemessen entlang dem Schlauch zwischen den Verbindern 208 und 209, gemäß Fig. 4 unterhalb von 17,15 cm sinkt, so wird ein Punkt erreicht, bei dem die erforderliche Rotationsgeschwindigkeit des Pumpenkopfes extrem hoch wird, wodurch die Lebensdauer des Schlauches entsprechend verkürzt wird und wo es bereits schwierig wird, den Schlauch um den Pumpenkopf mit einer entsprechend geringen Spannung zu erstrecken, um nur eine teilweise Occlusion an den Kontaktpunkten des Schlauches mit den Rollen zu erzielen. Bei Längen über 28 cm überschreitet die Länge des Schlauchpumpenabschnittes die Dimensionen des Pumpenkopfes und es wird überdurchschnittlich schwieriger, den Schlauchpumpenabschnitt unter Spannung um die Rollen des Pumpenkopfes zu dehnen mit einer ausreichenden Occlusion des Schlauches an den Druckpunkten durch die Rollen. Eine Schlauchwandstärke von zumindest 1,7 mm ist notwendig, um die Leckdichtigkeit des Schlauchpumpenabschnittes sicherzustellen, welcher kontinuierlichen und rasch wechselnden Ausdehnungen und Entspannungen unterworfen ist und auf diese Weise zu unnötiger Ermüdung und Zerreißen des Schlauchabschnittes bei geringeren Wandstärken neigt. Eine Schlauchwandstärke von mehr als 3,2 mm ergibt einen zu steifen Schlauchpumpenabschnitt für entsprechendes und geeignetes Zusammendrücken durch die Rollen. Wenn der Innendurchmesser des Schlauchpumpenabschnittes unter 4,5 mm absinkt, so entsteht eine ungeeignete Übereinstimmung zwischen der Schlauchgröße und den Anforderungen an die Rollengröße, mit dem Ergebnis, daß ein zu großer Bereich des Schlauches einer partiellen Occlusion unterworfen wird, wodurch eine entsprechende Reduktion der Pumpeneffizienz entsteht. Andererseits, wenn der Schlauchinnendurchmesser größer als 6,35 mm beträgt, bei einer vorhin genannten Wandstärke im Bereich zwischen 1,78 und 3,2 mm, so wird der Schlauchabschnitt bei höheren negativen Druckniveaus der Dialysatlösung sehr stark deformierbar, wodurch eine der Pumpeneffizienz bei derartigen hohen negativen Druckniveaus der Dialysatlösung entgegenstehender Effekt entsteht.

Schließlich umfaßt die peristaltische Dialysatpumpe gemäß der vorliegenden Erfindung einen mit dem Pumpenkopf gekuppelten Antrieb für die Dialysatlösungspumpe, um diese bei einer Geschwindigkeit im Bereich von 150-400 1/min zu drehen. Diese Grenzen werden durch die Dialysatlösungs-Flußrate, die für das Hemodialysesystem erforderlich ist, gesetzt. Um eine geeignete hohe Dialyseeffizienz zu erzielen, soll die Flußrate der Dialysatlösung im Dialysatlösungs-Kreislauf im Hemodialysesystem im Bereich von 450-550 ml/min liegen. Die Rotationsgeschwindigkeit des Dialysatpumpenan-

0004600

- 44 -

triebs ist eine Funktion des radialen Abstandes gemessen
zwischen einer bestimmten Pumpenachse und der fixen Achse
der Pumpenkopfbasis. Dies deshalb, weil das Pumpenflüssigkeitsvolumen im Schlauchabschnitt zwischen benachbarten
Rollen eine Funktion dieser radialen Distanz ist, sodaß
beim Sinken der radialen Distanz die Rotationsgeschwindigkeit des Antriebs für die Pumpe ansteigen muß, um die
Dialysatlösungs-Flußrate im Dialysatlösungs-Kreislauf auf
einem bestimmten Niveau zu halten. Für den oben genannten
radialen Abstand im Bereich von 1,27 bis 3,80 cm würde die
Rotationsgeschwindigkeit des Pumpenantriebes zur Aufrechterhaltung einer Dialysations-Flußrate in einem bestimmten
Bereich progressiv sinken, von ca. 400 1/min bei einem
radialen Abstand von 1,27 cm auf ca. 150 1/min bei einem
radialen Abstand vo 3,8 mm.

Vorzugsweise liegt die Länge des flexiblen elastischen
Schlauchpumpenabschnittes der erfindungsgemäßen Pumpe zwischen 17,8 und 25,4 cm und das Verhältnis der Wandstärke
zum Innendurchmesser des flexiblen elastischen Schlauchpumpenabschnittes im Bereich von 0,35 bis 0,40, um eine Überlastung des Dialysatlösungs-Pumpenantriebes zu vermeiden,
obwohl ein Schlauchpumpenabschnitt mit einem geeigneten
Deformationswiderstand beim Ansteigen des negativen Druckes
in der Dialysatlösung vorgesehen wird.

Fig. 5 ist ein Aufriß der Dialysatlösungspumpe und des
damit verbundenen Antriebs, wie sie in einem Hemodialysesystem gemäß der Fig. 2 verwendet wird und in Draufsicht in
Fig. 4 gezeigt ist. Die Systemelemente in Fig. 5 sind entsprechend der Fig. 4 numeriert.
Wie Fig. 5 zeigt, ist der Pumpenkopf in einem Behälter 200
befestigt, in welchem die Dialysatlösungseinlaßleitung 114
führt und an den oben beschriebenen Fitting 204 endet,
welches einen Flanschabschnitt 206 aufweist. Der drehbare
Pumpenkopf umfaßt ein Basiselement 201, welches zur Drehung
um eine fixe Achse mit mehreren am Umfang verteilten und
von einander getrennten Rollen 202a und 202c befestigt ist,
welche sich um ihre entsprechenden zur fixen Achse des Basisgliedes paralllelen Achsen unabhängig drehen. Das Basisglied 201 weist einen horizontalen Umfangsabschnitt 220 auf, auf dem die Rollen montiert sind, z.B. mit geeigneten Schrauben oder Bolzen. Die Rollen werden an ihren oberen Enden in ihrer Stellung durch eine Deckplatte 221 gehalten, mit der die Rollen in geeigneter Weise durch Schrauben oder Bolzen verbunden sind, welche eine unabhängige Drehung der Rollen um ihre eigenen Achsen erlauben. Die Rollen 202a und 202c haben jene eine im allgemeinen konvexe Profilfläche, um nur ein teilweises Verschließen des Schlauches an den Druckpunkten durch die Rollen vorzusehen. Die Rollen haben sich nach außen erstreckende Flanschabschnitte 222 und 223, um den Schlauchpumpenabschnitt in der richtigen Lage an der Rollenoberfläche während der Drehung aufzunehmen. Das Basiselement 201 des Pumpenkopfes besitzt eine erhabene Spindel 203, welche auf einer Welle 224 eines Elektroantriebsmotors 225 angeordnet ist, zu dem Strom mittels elektrischer Leitungen 226 und 227 geführt wird.

Fig. 6 ist ein Graph der Dialysatlösungs-Flußrate (Pumpenausstoß) in ml/min, gezeichnet als Funktion des negativen Drucks in mm Hg für eine Dialysatlösungspumpe gemäß der US-S.No. 720 672 (Kurve A) und einer Dialysatlösungspumpe gemäß der vorliegenden Erfindung (Kurve B). Die Dialysatlösungspumpe der Kurve A wurde konstruiert gemäß der Lehre der US-S.No. 720 672 und besitzt drei Rollen, jede mit einem Durchmesser von 12,7 mm mit einem radialen Abstand zwischen der Rollenachse und der fixen Achse des Basisgliedes von 19 mm. Der flexible elastische Schlauchpumpenabschnitt der bekannten Pumpe hat eine Länge von 16,8 cm, einen inneren Durchmesser von 4,75 mm und einen Außendurchmesser von 0,8 mm. Der mit dem Pumpenkopf verbundene Antrieb war für eine Geschwindigkeit von 400.1/min vorgesehen. Die Pumpe der Kurve B konstruiert gemäß der vorliegenden Erfindung, besaß vier Rollen, deren jede ein im allgemeinen konvexes Profil mit einem maximalen Durchmesser von 9,5 mm, mit einem radialen Abschnitt zwischen der Rollenachse und der fixen Achse des Basisgliedes von

sche flexible Schlauchpumpenabschnitt für diese Pumpe hat eine Länge von 22,2 cm, eine Wandstärke von 2,39 mm und einen Innendurchmesser von 6,35 mm. Der mit dem Pumpenkopf verbundene Antrieb war für eine Rotationsgeschwindigkeit von 200 1/min vorgesehen.

Wie die entsprechenden Kurven der Fig. 6 für die Dialysatlösungspumpe der vorliegenden Erfindung (Kurve B) zeigen, ergibt sich eine relativ konstante Ausflußrate, welche nur zwischen 490 und 570 ml/min variiert, über einen negativen Druckbereich von 0-350 mm Hg. Im Gegensatz dazu zeigte die bekannte Pumpe (Kurve A) eine drastische Reduktion der Ausflußrate der Dialysatlösung von ca. 560 ml/min bei 0 mm Hg negativen Druck hinunter bis ca. 150 ml/min bei einem negativen Druck von 300 mm Hg. Wie schon weiter oben besprochen, verursacht die totale Occlusion im Schlauchpumpenabschnitt an den Druckpunkten der Rollen zusammen mit den Rollenabständen und den Schlauchdimensionen des bekannten Pumpsystems eine sehr große Abflachung der Schlauchpumpenabschnitte zwischen benachbarten Rollen, sodaß der Anstieg des negativen Druckes die Schlauchabschnitte zwischen benachbarten Rollen sehr stark zusammendrückt, wodurch ein sehr starker Abfall der Pumpenausflußrate mit ansteigendem negativen Druck, wie im Graphen der Fig. 6 gezeigt ist, verursacht wird. Im Gegensatz dazu liegt die Ausflußrate der Dialysatlösung von der erfindungsgemäßen Pumpe innerhalb eines vergleichsweise schmalen Bereiches über den gesamten negativen Durckbereich, wie dies Kurve B zeigt. Wie Kurve B zeigt, ist der Abfall in der Flußrate vergleichsweise seicht bei ca. 60 mm Hg, gefolgt durch einen graduellen Anstieg der Ausflußrate bis ca. 200 mm Hg und einem wieder vergleichsweise seichten Abfall über 200 mm Hg negativen Druck. Diese verschiedenen Abschnitte der Kurve B können anhand des Grades der Occlusion und des Ausmaßes der Schlauchverformung über die aufeinanderfolgenden Bereiche des negativen Druck erklärt werden. Von 0-60 mm Hg Negativdruck resultiert der ansteigende negative Druck in einem progressiv ansteigenden niedrigen Deformationsniveau des Schlauchabschnittes zwischen benachbarten Rollen, sodaß die Dialysatlösungs-Flußrate der Pumpe über diesen negativen Druckbereich langsam abfällt. Über 60 mm Hg Negativdruck verursacht der ansteigende negative Druck ein progressives Ansteigen des Occlusionsgrades des Schlauches an den Stellen, an denen er durch die Rollen zusammengedrückt wird, woraus wieder ein Ansteigen der Pumpeneffizienz resultiert, welche das Abfallen des Flüssigkeitsvolumens in den Schlauchabschnitten zwischen benachbarten Rollen infolge des Ansteigens der Deformation mit ansteigendem negativem Druck mehr als ausgleicht, sodaß die Dialysatlösungs-Flußrate der Pumpe progressiv ansteigt, bis zu einem Wert von etwa 200 mm Hg negativem Druck. Über diesem negativen Druckniveau dominiert der Effekt des Abfalls des Flüssigkeitsvolumens des Schlauchabschnittes zwischen benachbarten Rollen der Pumpe leicht gegenüber der Verbesserung der Pumpeneffizienz in Verbindung mit dem Ansteigen des Bereiches der partialen Occlusion des Schlauches an den Druckpunkten der Rollen bei ansteigendem negativem Druck, sodaß die Kurve der Ausflußrate ein leichtes Absinken zeigt. Trotz dieses Wechsels in der Neigung über den negativen Druckbereich zeigt Kurve B nur eine Veränderung von 7,5 % von der mittleren Flußrate von 530 ml/min über den gesamten Bereich des negativen Drucks von 0-350 mm Hg. Demgegenüber zeigt das bekannte Pumpensystem einen scharfen Abfall in der Ausflußrate der Dialysatlösung von etwa 73 % über den negativen Druckbereich von 0-300 mm negativen Druck. Die Kurven in Fig. 6 zeigen deutliche die Verbesserung, welche mit der Pumpe gemäß der vorliegenden Erfindung relativ zur Pumpe der bekannten Art erzielt wird.

Fig. 7 zeigt eine Ausführungsform einer Rolle, wie sie in geeigneter Weise für die vorliegende Erfindung verwendet werden kann. Die Rolle hat eine tragende Fläche 300 mit der der Schlauch in der Art wie in Fig. 4 gezeigt, in Eingriff steht. Die tragende Fläche 300 hat im allgemeinen ein konvexes Profil, um den Schlauch an den Druckpunkten der Rolle nur teilweise zu verschließen. Mit der Bezeichnung im

- 48 -

0004600

allgemeinen konvexes Profil wird zum Ausdruck gebracht, daß die Flächenkontur der Tragfläche in einer radialen Ebene durch die Rotationsachse der Rolle im allgemeinen von konvexer Gestalt ist, wobei der Rollendurchmesser im Mittelabschnitt der Rolle 300 a größer ist, als der Rollendurchmesser an den Endabschnitten 300 b und 300 c der Rollentragfläche. Die Rolle hat einen maximalen Durchmesser gemessen in einer Ebene normal zur Drehachse der Rolle zwischen 6,35 und 19 mm und z.B. 9,5 mm. Wie gezeigt, ist die Rolle mit Endflanschen 301 und 302 versehen, welche dazu dienen, den Schlauch auf der Rollentragfläche 300 an Ort und Stelle zu halten. Die Rolle ist mit einer Achse 303 versehen, deren länglicher Achsabschnitt sich durch eine geeignete zentrale Passage des Rollenkörpers erstreckt. Die Achse 303 weist abgeflachte Endabschnitte 304 und 305 auf, welche die Rolle an dem länglichen Schaftabschnitt der Achse an ihrem Platz hält. Auf diese Weise können die Rollen auf der Pumpenkopfbasis befestigt sein, um sich unabhängig voneinander um zur fixen Achse der Basis parallele Achsen zu drehen, indem z.B. der abgeflachte Endabschnitt 305 der Achse mit der Pumpenkopfbasis mechanisch verbunden oder sonstwie verbunden ist.

In der Praxis können die Rollen aus einem geeigneten Konstruktionsmaterial bestehen. Bevorzugt werden Polymerwerkstoffe mit geringem Oberflächenreibungskoeffizient um den Abrieb des Schlauches durch die Rollentragfläche im Betrieb zu minimieren. Fig. 8 zeigt eine andere Ausführungsform einer Rolle, wie sie z.B. bei einer Rolle der vorliegenden Erfindung verwendet werden kann. Diese Rolle weist eine Tragfläche 306 mit einem im allgemeinen konvexen Profil auf. Die Rolle besitzt Endflansche 307 und 308, die den Schlauch auf der Tragfläche während des Betriebes in seiner Position halten. Eine Achse 309 erstreckt sich durch einen zentralen Durchgang im Rollenkörper und umfaßt abgeflachte Endabschnitte 310 und 311 zur Aufnahme der Rollen in einer Position auf dem Achsabschnitt der Achse. Die Rolle hat einen Maximaldurchmesser zwischen 6,35 und 19 mm. Die Rolle der Fig. 8 unterscheidet sich von jener der Fig. 7 in der Art, daß die erstere eine Tragflächenkontur aufweist, welche von zwei linearen schrägen Profilen gebildet ist, während letztere ein gleichmäßig konvex gebogenes Profil aufweist. Beide Ausführungsbeispiele der Rollen sehen ein im allgemeinen konvexes Profil für die Tragfläche vor, um den Schlauch an den Druckpunkten mit den Rollen nur teilweise zusammenzudrücken. Obwohl nur bevorzugte Ausführungen der Erfindung im Detail beschrieben wurden, ist es selbstverständlich, daß auch andere Ausführungsformen lediglich mit Modifikationen der geoffenbarten Eigenschaften ebenfalls im Bereich der Erfindung liegen.

0004600

Patentansprüche:

1. Hemodialyseapparat zur Behandlung von Blut zur Entfernung von Abfallverunreinigungen aus diesem, umfassend einen Dialysator, durch welchen das Abfallverunreinigungen enthaltende Blut und eine Dialysatlösung in indirekter Massenübertragungdialysebziehung geleitet werden, um die Abfallverunreinigungen vom Blut auf die Dialysatlösung zu übertragen, eine Einrichtung zur Übertragung des Abfallverunreinigungen enthaltenden Blutes von einem Patienten zum Dialysator und zur Rückführung des von Abfallverunreinigungen gesäuberten Blutes zum Patienten, eine Einrichtung zur Übertragung einer Dialysatlösung von einer Dialysatlösungsquelle zum Dialysator und eine Einrichtung zum Entleeren der mit Abfallverunreinigungen angereicherten Dialysatlösung vom Dialysator, wobei ein Dialysatlösungs-Kreislauf mit einem flexiblen elastischen Schlauchpumpenabschnitt gebildet wird, durch welchen die Dialysatlösung gepumpt wird, eine peristaltische Pumpe, mit einem drehbaren Pumpenkopf, welcher ein Basiselement umfaßt, welches um eine fixe Drehachse drehbar angeordnet ist und auf der mehrere über den Umfang verteilte Rollen befestigt sind, welche um entsprechende zur fixen Achse des Basiselementes parallelen Achsen unabhängig voneinander drehbar sind, einer Einrichtung zur Verankerung der Endabschnitte des flexiblen elastischen Schlauchpumpenabschnittes, sodaß sich der Schlauch unter Spannung um den Pumpenkopf herum erstreckt und gleichzeitig mit zumindest zwei der über den Umfang verteilten Rollen im Eingriff steht und von diesen zusammengedrückt wird, wobei diese Rollen zur Längsbewegung der Zusammendrückpunkte entlang des Schlauches während der Rotation des Pumpenkopfes angeordnet sind, um die Dialysatlösung durch den Schlauch weiterzubefördern, dadurch gekennzeichnet, daß die peristaltische Pumpe vier am Basisglied befestigte Rollen aufweist, deren jede zum teilweisen Zusammendrücken des Schlauches an den Druckpunkten durch die Rollen ein im allgemeinen konvexes Profil aufweist, mit einem Maximaldurchmesser im Bereich zwischen 6,35 und 19 mm, daß die Rollen gegenüber der benachbarten Rolle am Umfang um 90° versetzt angeordnet sind, wobei der radiale Abstand zwischen der Rollenachse und der fixen Achse des Basiselementes zwischen 12,7 und 38 mm beträgt, daß der flexible elastische Pumpenschlauchabschnitt eine Länge, gemessen entlang des Schlauches zwischen dessen verankerten Endabschnitten, von 17,15 bis 28 mm aufweist, bei einer Wandstärke von 1,7 bis 8,9 mm und einem inneren Durchmesser von 4,57 bis 8,9 mm, und daß mit dem Pumpenkopf Antriebsmittel verbunden sind für dessen Drehung bei einer Geschwindigkeit im Bereich von 50-400 1/min.

2. Apparat nach Anspruch 1, dadurch gekennzeichnet, daß die peristaltische Pumpe zum Betrieb in einem einstückigen Dialysebehältermodul montiert ist.

3. Apparat nach Anspruch 2, dadurch gekennzeichnet, daß ein Abschnitt des Dialysatlösungs-Kreislaufs in dem einstückigen Dialysebehältermodul angeordnet ist, wobei der Dialysatlösungs-Kreislaufabschnitt alternativ und lösbar entweder mit einem Dialysatlösungs-Proportioniersystem für den einfachen Durchfluß von Dialysatlösung durch den Dialysatlösungs-Kreislauf oder mit einem Dialysatlösungs-Speisekontainer zur chargenweisen Rückführung der Dialysatlösung durch den Dialysatlösungs-Kreislauf verbindbar ist.

4. Apparat nach Anspruch 3, dadurch gekennzeichnet, daß das Proportioniersystem eine Einrichtung aufweist, die für einen kontinuierlichen Wasserfluß bei erhöhter Temperatur vorgesehen ist zu einer Portioniereinrichtung, in der das Wasser und ein Dialysatkonzentrat in einem vorbestimmten Verhältnis miteinander vermischt werden, um die Dialysatlösung zu bilden, wobei die Einrichtung für den kontinuierlichen Wasserfluß bei erhöhter Temperatur eine Wasserquelle und eine Einrichtung zum Erwärmen des

0004600

Wassers von dieser Quelle auf die erhöhte Temperatur und eine Entlüftungseinrichtung zur Entfernung lösbarer Gase aus dem erwärmten Wasser vor dessen Durchfluß durch die Portioniereinrichtung umfaßt, wobei die Entlüftungseinrichtung

(a) einen ersten Behälter zur Aufnahme des erwärmten Wassers von der Erwärmungseinrichtung mit einer Entlüftungseinrichtung, welche das Entfernen von aus dem Wasser ausgeschiedenen Gasen zur Bildung eines teilweise entlüfteten Wassers zuläßt

(b) eine Vakuumpumpe

(c) eine Verbindungsleitung vom ersten Behälter zur Vakuumpumpe für die Zufuhr von teilweise entlüftetem Wasser vom ersten Behälter zur Vakuumpumpe

(d) eine einstellbare Fußbegrenzungseinrichtung in der Leitung zur Reduktion des Drucks des teilweise entlüfteten Wassers, welches durch die Leitung geführt wird zur weiteren Ausscheidung von Gasen und zur Bildung eines Niederdruckwassers, in dem abgeschiedenes Gas verteilt ist

(e) eine Leitung, die mit einem Ende am Auslaß der Vakuumpumpe zum Entleeren von Wasser mit höherem Druck mit im Wasser verteilten abgeschiedenem Gas abgeschlossen ist,

(f) einen zweiten Behälter, verbunden mit dem anderen Ende der Leitung (e) zur Aufnahme des Wassers mit höherem Druck mit einer Entlüftungsvorrichtung, die ein Entfernen des abgeschiedenen Gases aus dem Wasser mit höherem Druck zur Bildung eines endgültig entlüfteten Wassers zuläßt

(g) eine Einrichtung zur Übertragung des endgültig entlüfteten Wassers vom zweiten Reservoir zum Proportioniersystem umfaßt, wobei die Portioniereinrichtung mit dem Dialysat-Lösungskreislauf für dessen Speisung mit Dialysatlösung verbindbar ist.

5. Apparat nach Anspruch 4, dadurch gekennzeichnet, daß die Entlüftungseinrichtung einen Überlauf aufweist, zur Aufrechterhaltung eines Flüssigkeitsniveaus im ersten Behälter unterhalb eines vorherbestimmten Niveaus, ferner eine Leitung zur Verbindung des ersten Behälters mit dem zweiten Behälter zur Rückleitung eines Teils des endgültig entlüfteten Wassers vom zweiten Behälter zum ersten Behälter, um die Entfernung lösbarer Gase durch die Entlüftungseinrichtung zu erhöhen.

6. Vorrichtung nach Anspruch 4, dadurch gekennzeichnet, daß das Proportioniersystem zum Betrieb in einem einstückigen Behälter angebracht ist, als Dialysatlösungs-Speisemodul.

7. Vorrichtung nach Anspruch 2, dadurch gekennzeichnet, daß der Rest des Dialysatlösungs-Kreislaufes lösbar mit dem flexiblen elastischen Schlauchpumpenabschnitt verbunden ist.

8. Apparat nach Anspruch 1, dadurch gekennzeichnet, daß der flexible elastische Schlauchpumpenabschnitt aus Siliconelastomer gebildet ist.

9. Apparat nach Anspruch 8, dadurch gekennzeichnet, daß das Siliconelastomer eine Durometeranzeige von 40-60 aufweist.

10. Apparat nach Anspruch 1, dadurch gekennzeichnet, daß die Blutübertragungs- und Rückführeinrichtung einen Blutkreislauf aufweist, der mit dem Dialysator verbunden ist und einen flexiblen elastischen Schlauchpumpenabschnitt, durch den Blut gepumpt wird, aufweist, wobei weitere peristaltische Pumpen mit diesem elastischen flexiblen Schlauchpumpenabschnitt im Blutkreislauf verbunden sind.

11. Apparat nach Anspruch 1, dadurch gekennzeichnet, daß der Dialysatkreislauf mit der Dialysatlösungsquelle zur chargenweisen Zurückführung der Dialysatlösung durch den Dialysator einen geschlossenen Kreislauf bildet.

12. Apparat nach Anspruch 1, dadurch gekennzeichnet, daß die Dialysatlösungsquelle eine Portioniereinrichtung zur kontinuierlichen Mischung von Wasser aus einer bestimmten Quelle mit Dialysatkonzentrat zur Bildung der Dialysatlösung aufweist und daß die Dialysatlösung durch den Dialysatlösungs-Kreislauf in einem einzigen Durchgang fließen gelassen wird.

13. Apparat nach Anspruch 1, dadurch gekennzeichnet, daß der Dialysatlösungs-Kreislauf einen Abschnitt stromauf des Dialysators aufweist, mit dem eine Heizeinrichtung für die Dialysatlösung, eine Fühlvorrichtung für die Temperatur der Dialysatlösung stromab der Heizeinrichtung und zum Einstellen der Heizrate der Dialysatlösung durch die Heizeinrichtung, in Abhängigkeit von der gefühlten Temperatur zur Aufrechterhaltung eines vorherbestimmten Temperaturniveaus der Dialysatlösung und eine Einrichtung zur Messung der elektrolytischen Leitfähigkeit der Dialysatlösung verbunden sind.

14. Apparat nach Anspruch 13, dadurch gekennzeichnet, daß mit dem Dialysatlösungs-Kreislaufabschnitt stromab des Dialysators eine Einrichtung zur Auffindung von Blutleckagen verbunden ist.

15. Apparat nach Anspruch 13, dadurch gekennzeichnet, daß eine Einrichtung zur Umwandlung der erfühlten Temperatur der Dialysatlösung in ein übertragbares Signal vorgesehen ist, daß eine Einrichtung zur Übertragung des Temperaturfühlsignales vorgesehen ist, daß eine visuelle Anzeige mit der Temperaturfühlvorrichtung durch die Signalübertragungseinrichtung zur Anzeige der gefühlten Dialysatlösungstemperatur verbunden ist und daß eine Einrichtung zur Umwandlung der elektrolytischen Leitfähigkeit der Dialysatlösung in ein übertragbares Signal vorgesehen ist, daß eine Einrichtung zur Übertragung des elektrolytischen Leitfähigkeitssignals vorgesehen ist und daß eine visuelle Anzeige mit der elektrolytischen Leitfähigkeitsmeßvorrichtung durch die Leitfähigkeits-Signaleinrichtung zur Anzeige der gemessenen elektrolytischen
Leitfähigkeit der Dialysatlösung verbunden ist.

16. Hemodialyseapparat zur Behandlung von Blut, zur Entfernung von Abfallverunreinigungen aus diesem, umfassend:
einen Dialysator, durch welchen das Abfallverunreinigungen enthaltende Blut und eine Dialysatlösung in
indirekter Massenübertragungs-Dialysebeziehung zur
Übertragung der Abfallverunreinigungen vom Blut auf die
Dialysatlösung geleitet werden, eine Einrichtung zur
Übertragung des Abfallverunreinigungen enthaltenden
Blutes vom Patienten zum Dialysator, mit einem flexiblen elastischen Schlauchpumpenabschnitt, durch den
dasBlut gepumpt wird und mit einer Vorrichtung zur
Zurückleitung des von Abfallverunreinigungen gesäuberten Blutes zu dem Patienten unter Bildung eines Blutkreislaufes, eine Übertragungsvorrichtung für Dialysatlösung von einer Dialysatlösungsquelle zum Dialysator
und einer Vorrichtung zum Entleeren des Abfallverunreinigungen enthaltenden Dialysats vom Dialysator unter
Bildung eines Dialysatlösungskreislaufes mit einem
flexiblen elastischen Schlauchpumpenabschnitt, durch
welchen die Dialysatlösung gepumpt wird, zwei peristaltische Pumpen, jede mit einem drehbaren Pumpenkopf mit
einem Basiselement das zur Drehung um eine fixe Achse
angeordnet ist und mehrere um ihren Umfang verteilte
Rollen trägt, die unabhängig um entsprechende Achsen
parallel zur fixen Achse des Basiselements drehbar
sind, eine Einrichtung zur Verankerung der Endabschnitte des flexiblen elastischen Schlauchpumpenabschnittes
im Blutkreislauf und im Dialysatlösungs-Kreislauf,
sodaß der Schlauch in jedem Kreis unter Spannung um den
Pumpenkopf auf eine der beiden peristaltischen Pumpen
ausgedehnt wird und der Schlauch gleichzeitig mit
zumindest zwei der am Umfang verteilten Rollen der
peristaltischen Pumpe des Kreislaufes im Eingriff steht
und von diesem zusammengedrückt wird, wobei die Rollen
jeder peristaltischen Pumpe zur Längsbewegung der Druckpunkte entlang der angeschlossenen Schläuche während der Drehung des Pumpenkopfes für den Vorschub der Flüssigkeit durch den Schlauch angeordnet sind, dadurch gekennzeichnet, daß die peristaltische Pumpe für den Dialysatlösungs-Kreislauf vier an der Basis befestigte Rollen aufweist, deren jede ein im allgemeinen konvexes Profil besitzt, wobei der Schlauch an den Druckpunkten von den Rollen nur teilweise zusammengedrückt wird wobei die Rollen einen maximalen Durchmesser zwischen 6,35 und 19 mm aufweisen und gegeneinander bezüglich des Umfangs um 90° versetzt sind, wobei der radiale Abschnitt zwischen der Rollenachse und der fixen Achse des Basiselements zwischen 12,7 und 38 mm liegt, daß der flexible elastische Schlauchpumpenabschnitt im Dialysat-Kreislauf eine Länge gemessen längs des Schlauches zwischen den verankerten Endabschnitten von 17,14 bis 27,9 cm, bei einer Wandstärke von 1,7 bis 3,2 mm und einen Innendurchmesser von 4,5 und 8,9 mm beträgt und daß mit dem Pumpenkopf der peristaltischen Pumpe im Dialysatlösungs Kreislauf für deren Drehung mit einer Geschwindigkeit im Bereich von 150-400 1/min eine Antriebsvorrichtung gekuppelt ist.

17. Apparat nach Anspruch 16, dadurch gekennzeichnet, daß mit einem Abschnitt des Dialysatlösungs-Kreislaufes stromauf des Dialysators verbunden ist:

(a) eine Heizvorrichtung für die Dialysatlösung

(b) eine Einrichtung zur Fühlung der Temperatur der Dialysatlösung und Einstellung der Heizrate der Dialysatlösung durch die Heizvorrichtung in Abhängigkeit von der Temperaturfühlung zur Aufrechterhaltung eines vorbestimmten Temperaturniveaus der Dialysatlösung

(c) eine Einrichtung zur Umwandlung der Dialysatlösungstemperaturfühlung in ein übertragbares Signal

(d) eine Übertragungsvorrichtung für das Temperaturfühlsignal

(e) eine visuelle Anzeige, verbunden mit der Temperaturfühlvorrichtung durch die Signalübertragungseinrichtung zur Anzeige der gefühlten Dialysatlösungstemperatur,

(f) eine Einrichtung zur Messung der elektrolytischen Leitfähigkeit der Dialysatlösung

(g) eine Einrichtung zur Umwandlung der elektrolytischen Leitfähigkeit der Dialysatlösung in ein übertragbares Signal

(h) eine Übertragungsvorrichtung für das elektrolytische Leitfähigkeitssignal und

(i) eine visuelle Anzeige verbunden mit elektrolytischen Leitfähigkeitsmeßvorrichtungen durch die Leitfähigkeitssignalübertragungsvorrichtung zur Anzeige der festgestellten elektrolytischen Leitfähigkeit der Dialysatlösung und daß ein Abschnitt des Dialysatlösungs-Kreislaufes stromab des Dialysators mit einer Einrichtung (j) zur Feststellung von Blutleckage verbunden ist wobei die zweifache peristaltische Pumpe die Ankervorrichtung, die Pumpenantriebsvorrichtungen und die Einrichtungen (a) bis (j), verbunden mit dem Dialysatlösungsflußkreislauf im Betrieb in einem einstückigen Behälter angeordnet ist.

18. Vorrichtung nach Anspruch 1, dadurch gekennzeichnet, daß der flexible elastische Schlauchpumpenabschnitt eine Länge von 17,78 bis 25,4 cm aufweist.

19. Vorrichtung nach Anspruch 1, dadurch gekennzeichnet, daß das Verhältnis von Wandstärke zu Innendurchmesser des flexiblen elastischen Schlauchpumpenabschnittes im Bereich zwischen 0,35 und 0,4 liegt.

FIG. 1

0004600

FIG. 2

0004600

FIG. 3

129 130 127 128 166 165 163 164 162 159 160 167 168 158 157 156 155 161 153 154 152 151 117 150 126

FIG. 4

FIG. 7

FIG. 8

0004600

⁴/₅

FIG. 5

FIG. 6